# EUROPEAN PATENT APPLICATION

(11) **EP 4 796 114 A1**
(43) Date of publication of application: **26.08.2026**
(21) Application number: 26160367.4
(22) Date of filing: 24.02.2026
(51) Int. Cl.: A61K 31/519, A61P 35/00

(54) **NOVEL THIAZOLOPYRIDINE DERIVATIVES AS AROMATASE INHIBITORS FOR THE TREATMENT OF BREAST CANCER**

(30) Priority: 24.02.2025 TR 2025002225
(71) Applicant: ANADOLU UNIVERSITESI STRATEJI GELISTIRME DAIRE BASKANLIGI, 26470 Eskisehir (TR)
(72) Inventor: OZDEMIR, Ahmet, 26180 Eskisehir (TR); ALTINTOP, Mehlika Dilek, 26200 Eskisehir (TR); SAGLIK OZKAN, Begum Nurpelin, 26120 Eskisehir (TR)
(74) Representative: Sevinç, Cenk

(57) **Abstract**

The invention is related to the development of novel thiazolo[5,4-b]pyridine derivatives as anticancer compounds for use in the treatment of breast cancer. These compounds target estrogen-dependent cancer types through aromatase inhibition while providing selective cytotoxic effects against human breast adenocarcinoma (MCF-7) cells without causing damage to healthy (NIH/3T3) cells. Being easily and economically synthesized, these compounds aim to reduce side-effect and resistance issues encountered in current therapies.

## Description

### Technical Field of the Invention

The invention is related to thiazolo[5,4-*b*]pyridine derivatives as anticancer compounds to be used in the treatment of breast cancer, and to the synthesis method of these compounds and *in vitro* studies on their anticancer activity thereof. These compounds exhibit selective cytotoxic effects on human breast adenocarcinoma (MCF-7) cells without showing toxic effects on healthy (NIH/3T3) cells, and are effective as aromatase inhibitors. The chemical synthesis of the compounds is easy and economical, and it is also aimed to reduce problems such as side effects and resistance in treatment.

### State of the Art

The chemical and pharmaceutical industry has great importance, particularly in the design and development of anticancer drugs. Breast cancer stands out as one of the most common cancers among women worldwide and one of the leading causes of cancer-related deaths. In this context, intensive research and development activities are being carried out on antineoplastic drug candidates that can be used in the treatment of breast cancer. In this field, aromatase enzyme inhibitors used particularly in the treatment of estrogen-dependent tumours are widely preferred in targeted therapies. Steroidal (formestane, exemestane) and non-steroidal (anastrozole, letrozole) aromatase inhibitors aim to control tumour growth by suppressing estrogen production.

Although existing aromatase inhibitors have achieved a certain level of success in terms of efficacy, they have some important limitations. First of all, resistance to treatment is frequently observed during long-term use of these compounds. The resistance observed during the treatment process leads to a decrease in patients' response to treatment and the need for alternative treatment options. In addition, side effects of existing drugs such as arthralgia, insomnia, headache and nausea, which negatively affect patients' quality of life, are frequently reported. This situation constitutes a serious problem, especially in patients requiring long-term treatment.

Although various studies have been conducted in the literature on molecular modifications of aromatase inhibitors used in the treatment of breast cancer, innovative approaches in this field are limited. In particular, the identification of new chemical classes exhibiting inhibitory effects on the aromatase enzyme represents a significant gap in the current literature. Studies on targeting the aromatase enzyme have generally focused on the modification of known steroidal or non-steroidal structures. However, these approaches remain insufficient in terms of increasing molecular diversity and developing more tolerable compounds.

Among the commonly used aromatase inhibitors are anastrozole, letrozole and exemestane. During the use of these drugs, side effects such as joint and muscle pain, decreased bone mineral density and increased risk of osteoporosis may be observed. In addition, menopause-like symptoms such as hot flashes, night sweats and vaginal dryness may also occur. A decrease in estrogen levels may lead to negative changes in lipid profiles, thereby increasing the risk of cardiovascular diseases. During long-term use, tumour cells may develop resistance to aromatase inhibitors, which reduces treatment efficacy. Considering these side effects and limitations, it is important that patients are regularly monitored and necessary precautions are taken during the use of aromatase inhibitors.

Due to limitations and inadequacies of the solutions in the state of the art, such as the development of resistance to aromatase inhibitors used in treatment, the inability to provide appropriate treatment for this reason, the presence of side effects such as arthralgia, insomnia, headache and nausea, and the insufficiency of molecular diversity in terms of developing tolerable compounds, it has become necessary to develop new aromatase inhibitors to be used in the treatment of breast cancer.

### Brief Description and Aims of the Invention

The invention is related to new-generation thiazolo[5,4-*b*]pyridine derivatives as anticancer compounds for the treatment of breast cancer. These compounds are effective on human breast adenocarcinoma (MCF-7) cells by targeting the aromatase enzyme, while exhibiting selective cytotoxic properties without harming healthy cells. With these compounds, which can be synthesized by simple and low-cost methods, it is aimed to minimize problems such as side effects and resistance frequently encountered in existing treatments.

One aim of the invention is to obtain anticancer compounds exhibiting selective cytotoxic effects. Thus, by reducing the general toxicity problems caused by chemotherapy, it is aimed to provide targeted treatment of breast cancer and to increase patients' quality of life. At the same time, these compounds offer a more tolerable option in the treatment of breast cancer.

Another aim of the invention is to obtain anticancer compounds that reduce the risk of treatment resistance. Providing an effective solution for breast cancer cases resistant to aromatase enzyme inhibitors is an important aim of the invention. The distinct chemical structures of the new compounds provide an alternative strategy to overcome drug resistance. This approach constitutes a significant advantage in addressing the challenges encountered in the treatment of breast cancer.

In addition, the invention aims to obtain economical and easily obtainable aromatase inhibitors. The thiazolo[5,4-b]pyridine derivatives developed within the scope of the invention are produced by easy and low-cost synthesis methods. This accelerates the drug production process and offers economic advantages at both local and global scales.

### Description of the Figures

**Figure 1****.** IR spectrum of 1-(4-Chlorophenyl)-2-(thiazolo[5,4-*b*]pyridin-2-ylthio)ethan-1-one (Compound 1)
**Figure 2****.** ¹H-NMR spectrum of 1-(4-Chlorophenyl)-2-(thiazolo[5,4-*b*]pyridin-2-ylthio)ethan-1-one (Compound 1)
**Figure 3****.** ¹³C-NMR spectrum of 1-(4-Chlorophenyl)-2-(thiazolo[5,4-*b*]pyridin-2-ylthio)ethan-1-one (Compound 1)
**Figure 4****.** HRMS spectrum of 1-(4-Chlorophenyl)-2-(thiazolo[5,4-*b*]pyridin-2-ylthio)ethan-1-one (Compound 1)
**Figure 5****.** IR spectrum of 1-Phenyl-2-(thiazolo[5,4-*b*]pyridin-2-ylthio)ethan-1-one (Compound 2)
**Figure 6****.** ¹H-NMR spectrum of 1-Phenyl-2-(thiazolo[5,4-*b*]pyridin-2-ylthio)ethan-1-one (Compound 2)
**Figure 7****.** ¹³C-NMR spectrum of 1-Phenyl-2-(thiazolo[5,4-*b*]pyridin-2-ylthio)ethan-1-one (Compound 2)
**Figure 8****.** HRMS spectrum of 1-Phenyl-2-(thiazolo[5,4-*b*]pyridin-2-ylthio)ethan-1-one (Compound 2)
**Figure 9****.** IR spectrum of 1-(4-Fluorophenyl)-2-(thiazolo[5,4-*b*]pyridin-2-ylthio)ethan-1-one (Compound 3)
**Figure 10****.** ¹H-NMR spectrum of 1-(4-Fluorophenyl)-2-(thiazolo[5,4-*b*]pyridin-2-ylthio)ethan-1-one (Compound 3)
**Figure 11****.** ¹³C-NMR spectrum of 1-(4-Fluorophenyl)-2-(thiazolo[5,4-*b*]pyridin-2-ylthio)ethan-1-one (Compound 3)
**Figure 12****.** HRMS spectrum of 1-(4-Fluorophenyl)-2-(thiazolo[5,4-*b*]pyridin-2-ylthio)ethan-1-one (Compound 3)
**Figure 13****.** IR spectrum of 1-(4-Trifluoromethylphenyl)-2-(thiazolo[5,4-*b*]pyridin-2-ylthio)ethan-1-one (Compound 4)
**Figure 14****.** ¹H-NMR spectrum of 1-(4-Trifluoromethylphenyl)-2-(thiazolo[5,4-*b*]pyridin-2-ylthio)ethan-1-one (Compound 4)
**Figure 15****.** ¹³C-NMR spectrum of 1-(4-Trifluoromethylphenyl)-2-(thiazolo[5,4-*b*]pyridin-2-ylthio)ethan-1-one (Compound 4)
**Figure 16****.** HRMS spectrum of 1-(4-Trifluoromethylphenyl)-2-(thiazolo[5,4-*b*]pyridin-2-ylthio)ethan-1-one (Compound 4)
**Figure 17****.** IR spectrum of 1-(4-Cyanophenyl)-2-(thiazolo[5,4-*b*]pyridin-2-ylthio)ethan-1-one (Compound 5)
**Figure 18****.** ¹H-NMR spectrum of 1-(4-Cyanophenyl)-2-(thiazolo[5,4-*b*]pyridin-2-ylthio)ethan-1-one (Compound 5)
**Figure 19****.** ¹³C-NMR spectrum of 1-(4-Cyanophenyl)-2-(thiazolo[5,4-*b*]pyridin-2-ylthio)ethan-1-one (Compound 5)
**Figure 20****.** HRMS spectrum of 1-(4-Cyanophenyl)-2-(thiazolo[5,4-*b*]pyridin-2-ylthio)ethan-1-one (Compound 5)
**Figure 21****.** IR spectrum of 1-(4-Methylphenyl)-2-(thiazolo[5,4-*b*]pyridin-2-ylthio)ethan-1-one (Compound 6)
**Figure 22****.** ¹H-NMR spectrum of 1-(4-Methylphenyl)-2-(thiazolo[5,4-*b*]pyridin-2-ylthio)ethan-1-one (Compound 6)
**Figure 23****.** ¹³C-NMR spectrum of 1-(4-Methylphenyl)-2-(thiazolo[5,4-*b*]pyridin-2-ylthio)ethan-1-one (Compound 6)
**Figure 24****.** HRMS spectrum of 1-(4-Methylphenyl)-2-(thiazolo[5,4-*b*]pyridin-2-ylthio)ethan-1-one (Compound 6)
**Figure 25****.** IR spectrum of 1-(4-Methoxyphenyl)-2-(thiazolo[5,4-*b*]pyridin-2-ylthio)ethan-1-one (Compound 7)
**Figure 26****.** ¹H-NMR spectrum of 1-(4-Methoxyphenyl)-2-(thiazolo[5,4-*b*]pyridin-2-ylthio)ethan-1-one (Compound 7)
**Figure 27****.** ¹³C-NMR spectrum of 1-(4-Methoxyphenyl)-2-(thiazolo[5,4-*b*]pyridin-2-ylthio)ethan-1-one (Compound 7)
**Figure 28****.** HRMS spectrum of 1-(4-Methoxyphenyl)-2-(thiazolo[5,4-b]pyridin-2-ylthio)ethan-1-one (Compound 7)
**Figure 29****.** IR spectrum of 1-(4-Nitrophenyl)-2-(thiazolo[5,4-*b*]pyridin-2-ylthio)ethan-1-one (Compound 8)
**Figure 30****.** ¹H-NMR spectrum of 1-(4-Nitrophenyl)-2-(thiazolo[5,4-*b*]pyridin-2-ylthio)ethan-1-one (Compound 8)
**Figure 31****.** ¹³C-NMR spectrum of 1-(4-Nitrophenyl)-2-(thiazolo[5,4-*b*]pyridin-2-ylthio)ethan-1-one (Compound 8)
**Figure 32****.** HRMS spectrum of 1-(4-Nitrophenyl)-2-(thiazolo[5,4-*b*]pyridin-2-ylthio)ethan-1-one (Compound 8)
**Figure 33****.** IR spectrum of 1-(4-Methylsulfonylphenyl)-2-(thiazolo[5,4-*b*]pyridin-2-ylthio)ethan-1-one (Compound 9)
**Figure 34****.** ¹H-NMR spectrum of 1-(4-Methylsulfonylphenyl)-2-(thiazolo[5,4-*b*]pyridin-2-ylthio)ethan-1-one (Compound 9)
**Figure 35****.** ¹³C-NMR spectrum of 1-(4-Methylsulfonylphenyl)-2-(thiazolo[5,4-*b*]pyridin-2-ylthio)ethan-1-one (Compound 9)
**Figure 36****.** HRMS spectrum of 1-(4-Methylsulfonylphenyl)-2-(thiazolo[5,4-*b*]pyridin-2-ylthio)ethan-1-one (Compound 9)
**Figure 37****.** IR spectrum of 1-(Naphthalen-2-yl)-2-(thiazolo[5,4-*b*]pyridin-2-ylthio)ethan-1-one (Compound 10)
**Figure 38****.** ¹H-NMR spectrum of 1-(Naphthalen-2-yl)-2-(thiazolo[5,4-*b*]pyridin-2-ylthio)ethan-1-one (Compound 10)
**Figure 39****.** ¹³C-NMR spectrum of 1-(Naphthalen-2-yl)-2-(thiazolo[5,4-*b*]pyridin-2-ylthio)ethan-1-one (Compound 10)
**Figure 40****.** HRMS spectrum of 1-(Naphthalen-2-yl)-2-(thiazolo[5,4-*b*]pyridin-2-ylthio)ethan-1-one (Compound 10)
**Figure 41****.** IR spectrum of 1-(1,3-Benzodioxol-5-yl)-2-(thiazolo[5,4-*b*]pyridin-2-ylthio)ethan-1-one (Compound 11)
**Figure 42****.** ¹H-NMR spectrum of 1-(1,3-Benzodioxol-5-yl)-2-(thiazolo[5,4-*b*]pyridin-2-ylthio)ethan-1-one (Compound 11)
**Figure 43****.** ¹³C-NMR spectrum of 1-(1,3-Benzodioxol-5-yl)-2-(thiazolo[5,4-*b*]pyridin-2-ylthio)ethan-1-one (Compound 11)
**Figure 44****.** HRMS spectrum of 1-(1,3-Benzodioxol-5-yl)-2-(thiazolo[5,4-*b*]pyridin-2-ylthio)ethan-1-one (Compound 11)

### Detailed Description of the Invention

The invention relates to thiazolo[5,4-b]pyridine derivatives represented by Formula X to be used in the treatment of breast cancer by reducing estrogen levels through aromatase inhibition.

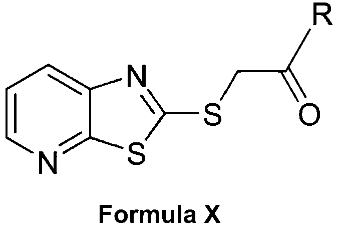

Here, any one of the formulas indicated below as R=R₁-R₁₁ is added in place of the group indicated by R.

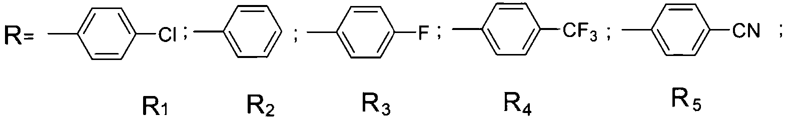

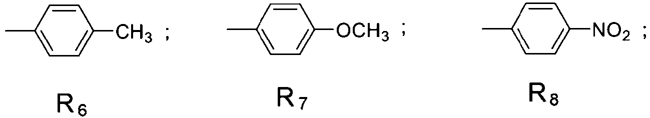

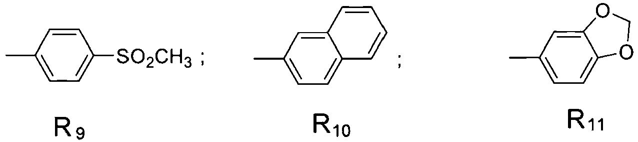

Thiazolo[5,4-b]pyridine derivatives (Compound 1 - Compound 11), which provide therapeutic benefit in breast cancer by reducing estrogen levels through aromatase inhibition, are obtained by adding any one of the R₁-R₁₁ groups in place of the R group present in said Formula X structure, respectively. Said compounds (Compound 1 - Compound 11) are as follows;
- 1-(4-Chlorophenyl)-2-(thiazolo[5,4-*b*]pyridin-2-ylthio)ethan-1-one (Compound 1) or
- 1-Phenyl-2-(thiazolo[5,4-*b*]pyridin-2-ylthio)ethan-1-one (Compound 2) or
- 1-(4-Fluorophenyl)-2-(thiazolo[5,4-*b*]pyridin-2-ylthio)ethan-1-one (Compound 3) or
- 1-(4-Trifluoromethylphenyl)-2-(thiazolo[5,4-*b*]pyridin-2-ylthio)ethan-1-one (Compound 4) or
- 1-(4-Cyanophenyl)-2-(thiazolo[5,4-*b*]pyridin-2-ylthio)ethan-1-one (Compound 5) or
- 1-(4-Methylphenyl)-2-(thiazolo[5,4-*b*]pyridin-2-ylthio)ethan-1-one (Compound 6) or
- 1-(4-Methoxyphenyl)-2-(thiazolo[5,4-*b*]pyridin-2-ylthio)ethan-1-one (Compound 7) or
- 1-(4-Nitrophenyl)-2-(thiazolo[5,4-*b*]pyridin-2-ylthio)ethan-1-one (Compound 8) or
- 1-(4-Methylsulfonylphenyl)-2-(thiazolo[5,4-*b*]pyridin-2-ylthio)ethan-1-one (Compound 9) or
- 1-(Naphthalen-2-yl)-2-(thiazolo[5,4-*b*]pyridin-2-ylthio)ethan-1-one (Compound 10) or
- 1-(1,3-Benzodioxol-5-yl)-2-(thiazolo[5,4-*b*]pyridin-2-ylthio)ethan-1-one (Compound 11).

The compound 1-(4-Chlorophenyl)-2-(thiazolo[5,4-*b*]pyridin-2-ylthio)ethan-1-one (Compound 1), obtained when the R group in said Formula X is R=R₁, is represented by Formula 1.

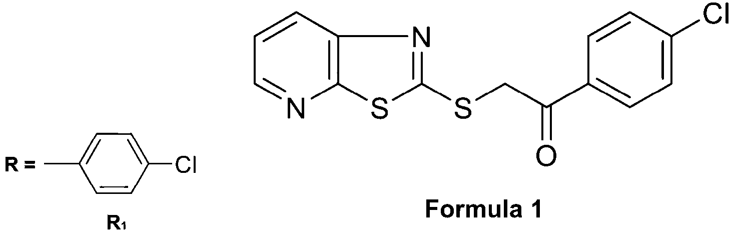

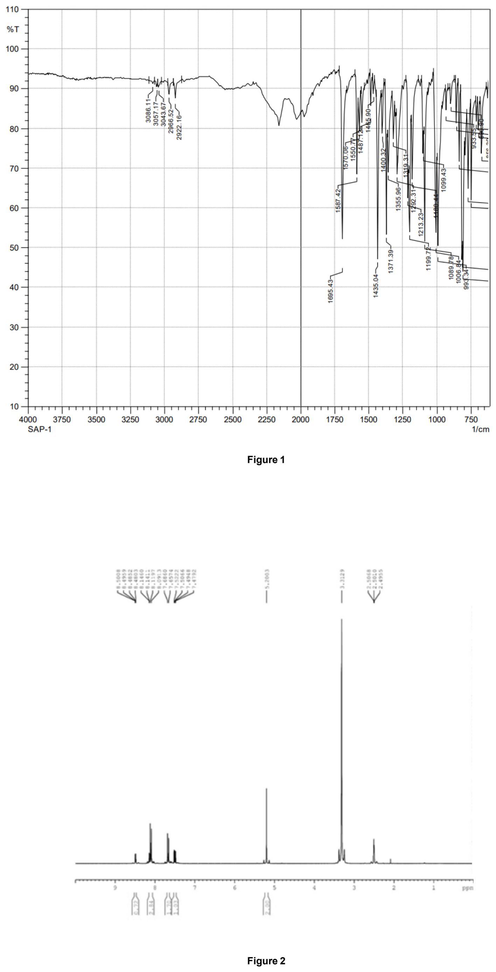

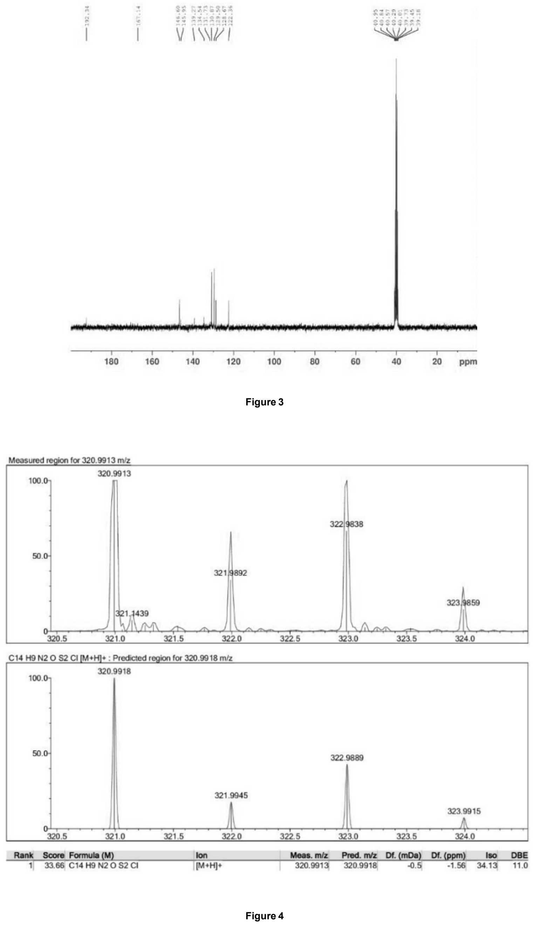

The characteristic properties of Compound 1 are given below.
- The melting point is 132-134°C.
- IR spectrum, IR vₘₐₓ (cm⁻¹): 3086.11, 3057.17, 3043.67 (aromatic C-H stretching bands), 2966.52, 2922.16 (aliphatic C-H stretching bands), 1695.43 (C=O stretching band), 1587.42, 1570.06, 1550.77, 1487.12, 1465.90 (C=C and C=N stretching bands), 1435.04, 1400.32, 1371.39, 1355.96, 1319.31, 1292.31, 1213.23, 1199.72, 1180.44, 1099.43, 1089.78, 1006.84 (C-H bending, C-N stretching and aromatic C-H in-plane bending bands), 993.34, 933.55, 896.90, 856.39, 835.18, 813.96, 808.17, 771.53, 746.45, 709.80, 694.37, 671.23 (aromatic C-H out-of-plane bending bands and C-S stretching band) (Figure 1).
- ¹H-NMR spectrum, ¹H-NMR (300 MHz, DMSO-d₆) δ (ppm): 5.20 (s, 2H), 7.50 (dd, *J* = 4.68, 8.22 Hz, 1H), 7.67 (d, *J* = 8.58 Hz, 2H), 8.11 (d, *J* = 8.52 Hz, 2H), 8.14 (d, *J* = 1.47 Hz, 1H), 8.49 (dd, *J* = 1.47, 4.68 Hz, 1H) (Figure 2).
- ¹³C-NMR spectrum, ¹³C-NMR (75 MHz, DMSO-d₆) δ (ppm): 40.95 (CH₂), 122.36 (CH), 128.67 (CH), 129.50 (2CH), 130.87 (2CH), 131.73 (C), 134.54 (C), 139.27 (C), 145.95 (C), 146.60 (CH), 167.14 (C), 192.34 (C) (Figure 3).
- HRMS spectrum, HRMS (ESI) *(m*/*z):* [M+H]⁺ calculated for C₁₄H₉ClN₂OS₂: 320.9918, found: 320.9913 (Figure 4).

The synthesis method of Compound 1 comprises the process steps of;
**a.** adding thiazolo[5,4-*b*]pyridin-2(1*H*)-thione, 2-bromo-4'-chloroacetophenone and a potassium carbonate catalyst in equivalent amounts,
**b.** adding 20 mL of acetone to the mixture,
**c.** completing the reaction by stirring the acetone-added mixture at room temperature for 6 hours,
**d.** subsequently evaporating acetone, which is the solvent of the reaction medium, and washing the resulting solid product with water, and
**e.** drying the washed solid product and crystallising it from ethanol.

The compound 1-Phenyl-2-(thiazolo[5,4-*b*]pyridin-2-ylthio)ethan-1-one (Compound 2), obtained when the R group in said Formula X is R=R₂, is represented by Formula 2.

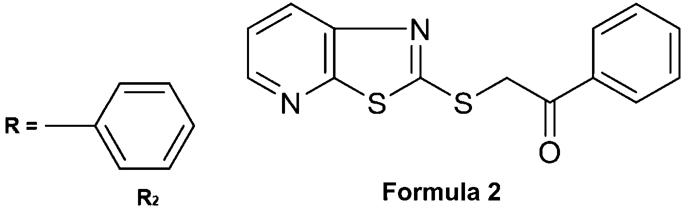

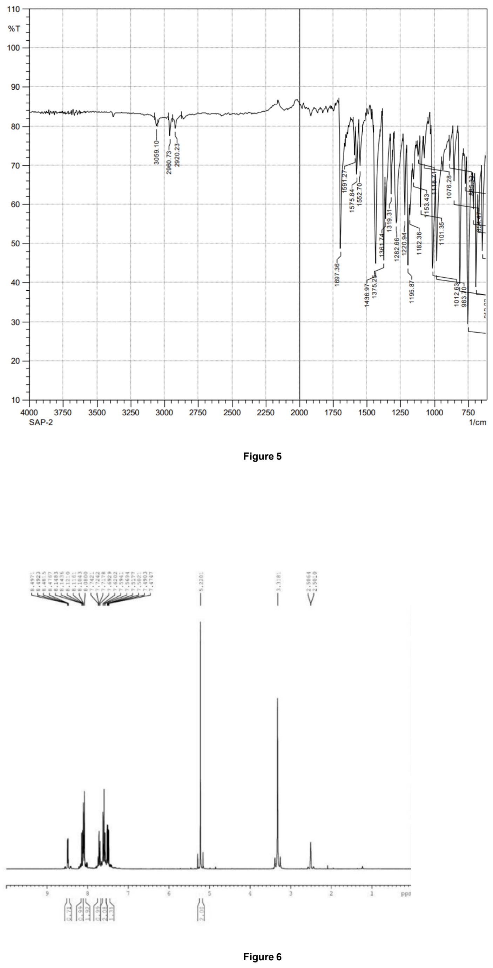

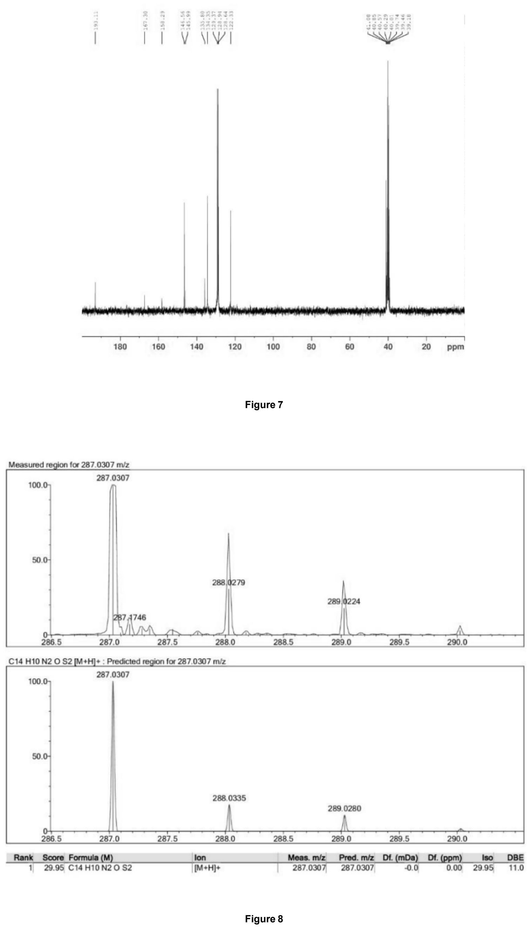

The characteristic properties of Compound 2 are given below.
- The melting point is 98-100°C.
- IR spectrum, IR vₘₐₓ (cm⁻¹): 3059.10 (aromatic C-H stretching band), 2960.73, 2920.23 (aliphatic C-H stretching bands), 1697.36 (C=O stretching band), 1591.27, 1575.84, 1552.70 (C=C and C=N stretching bands), 1436.97, 1375.25, 1361.74, 1319.31, 1282.66, 1220.94, 1195.87, 1182.36, 1153.43, 1118.71, 1101.35, 1076.28, 1012.63 (C-H bending, C-N stretching and aromatic C-H in-plane bending bands), 983.70, 885.33, 854.47, 812.03, 769.60, 748.38, 711.73, 690.52, 673.16, 644.22 (aromatic C-H out-of-plane bending bands and C-S stretching band) (Figure 5).
- ¹H-NMR spectrum, ¹H-NMR (300 MHz, DMSO-d₆) δ (ppm): 5.22 (s, 2H), 7.50 (dd, *J* = 4.68, 8.22 Hz, 1H), 7.60 (t, *J* = 7.62 Hz, 2H), 7.69 (d, *J* = 8.43 Hz, 1H), 8.09 (d, *J* = 7.29 Hz, 2H), 8.13 (dd, *J* = 1.41, 8.19 Hz, 1H), 8.49 (dd, *J* = 1.44, 4.68 Hz, 1H) (Figure 6).
- ¹³C-NMR spectrum, ¹³C-NMR (75 MHz, DMSO-d₆) δ (ppm): 41.08 (CH₂), 122.33 (CH), 128.64 (CH), 128.94 (2CH), 129.37 (2CH), 134.35 (CH), 135.80 (C), 145.99 (C), 146.56 (CH), 158.29 (C), 167.30 (C), 193.11 (C) (Figure 7).
- HRMS spectrum, HRMS (ESI) *(m*/*z):* [M+H]⁺ calculated for C₁₄H₁₀N₂OS₂: 287.0307, found: 287.0307 (Figure 8).

The synthesis method of Compound 2 comprises the process steps of;
**a.** adding thiazolo[5,4-*b*]pyridin-2(1*H*)-thione, 2-bromoacetophenone and a potassium carbonate catalyst in equivalent amounts,
**b.** adding 20 mL of acetone to the mixture,
**c.** completing the reaction by stirring the acetone-added mixture at room temperature for 6 hours,
**d.** subsequently evaporating acetone, which is the solvent of the reaction medium, and washing the resulting solid product with water, and
**e.** drying the washed solid product and crystallising it from ethanol.

The compound 1-(4-Fluorophenyl)-2-(thiazolo[5,4-*b*]pyridin-2-ylthio)ethan-1-one (Compound 3), obtained when the R group in said Formula X is R=R₃, is represented by Formula 3.

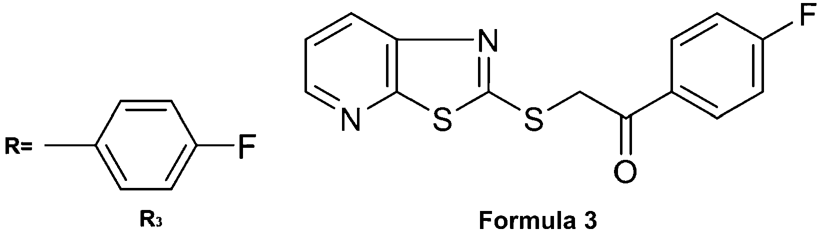

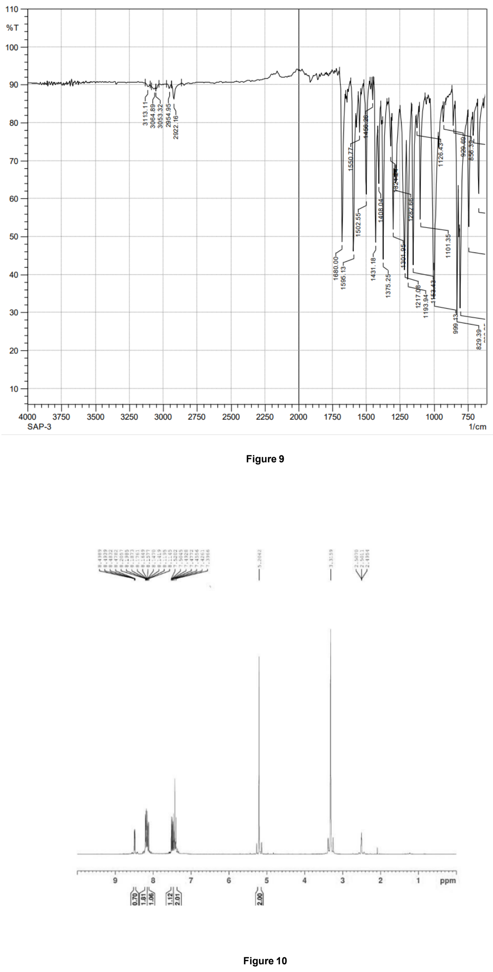

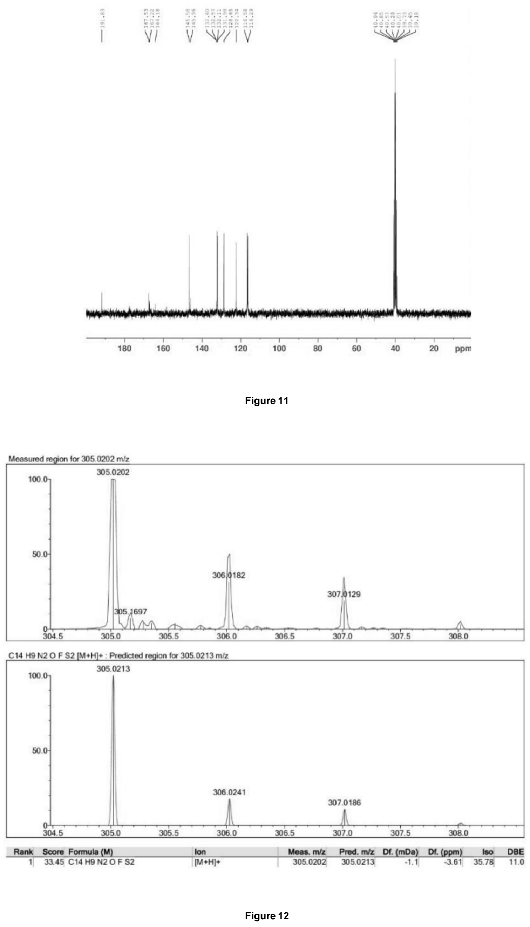

The characteristic properties of Compound 3 are given below.
- The melting point is 102-104°C.
- IR spectrum, IR vₘₐₓ (cm⁻¹): 3113.11, 3064.89, 3053.32 (aromatic C-H stretching bands), 2954.95, 2922.16 (aliphatic C-H stretching bands), 1680.00 (C=O stretching band), 1595.13, 1550.77, 1502.55, 1456.26 (C=C and C=N stretching bands), 1431.18, 1408.04, 1375.25, 1321.24, 1301.95, 1282.66, 1217.08, 1193.94, 1153.43, 1126.43, 1101.35 (C-H bending, C-N stretching and aromatic C-H in-plane bending bands), 999.13, 929.69, 856.39, 829.39, 806.25, 744.52, 707.88, 671.23 (aromatic C-H out-of-plane bending bands and C-S stretching band) (Figure 9).
- ¹H-NMR spectrum, ¹H-NMR (300 MHz, DMSO-d₆) δ (ppm): 5.20 (s, 2H), 7.43 (t, *J* = 8.85 Hz, 2H), 7.50 (dd, *J* = 4.71, 8.22 Hz, 1H), 8.13 (dd, *J* = 1.53, 8.25 Hz, 1H), 8.18 (td, *J* = 6.12, 3.36, 2.16 Hz, 2H), 8.49 (dd, J = 1.50, 4.71 Hz, 1H) (Figure 10).
- ¹³C-NMR spectrum, ¹³C-NMR (75 MHz, DMSO-d₆) δ (ppm): 40.94 (CH₂), 116.44 (d, *J* = 21.95 Hz, 2CH), 122.34 (CH), 128.65 (CH), 132.05 (d, *J* = 9.58 Hz, 2CH), 132.59 (d, *J* = 2.59 Hz, C), 145.96 (C), 146.58 (CH), 164.18 (C), 167.22 (C), 167.53 (C), 191.83 (C) (Figure 11).
- HRMS spectrum, HRMS (ESI) *(m*/*z):* [M+H]⁺ calculated for C₁₄H₉FN₂OS₂: 305.0213, found: 305.0202 (Figure 12).

The synthesis method of Compound 3 comprises the process steps of;
**a.** adding thiazolo[5,4-*b*]pyridin-2(1*H*)-thione, 2-bromo-4'-fluoroacetophenone and a potassium carbonate catalyst in equivalent amounts,
**b.** adding 20 mL of acetone to the mixture,
**c.** completing the reaction by stirring the acetone-added mixture at room temperature for 6 hours,
**d.** subsequently evaporating acetone, which is the solvent of the reaction medium, and washing the resulting solid product with water, and
**e.** drying the washed solid product and crystallising it from ethanol.

The compound 1-(4-Trifluoromethylphenyl)-2-(thiazolo[5,4-*b*]pyridin-2-ylthio)ethan-1-one (Compound 4), obtained when the R group in said Formula X is R=R₄, is represented by Formula 4.

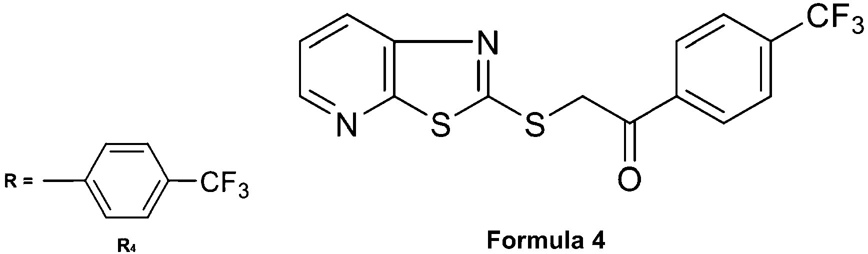

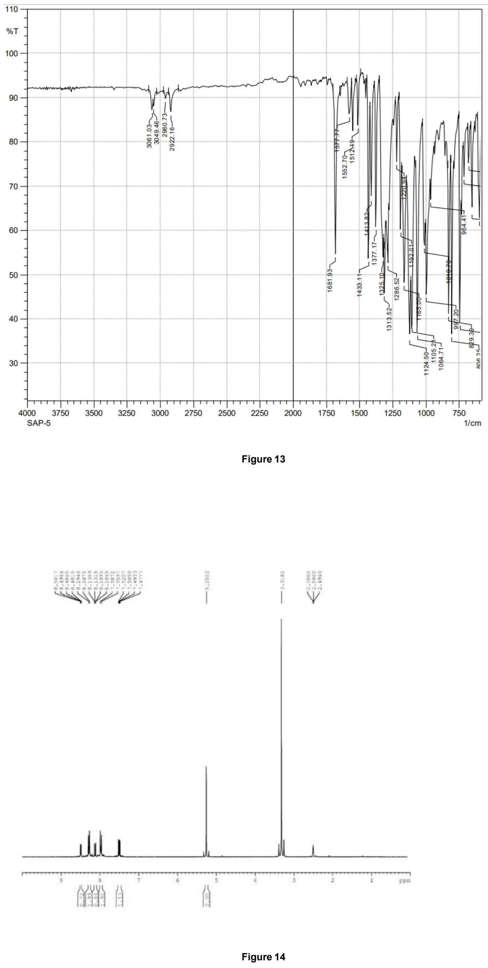

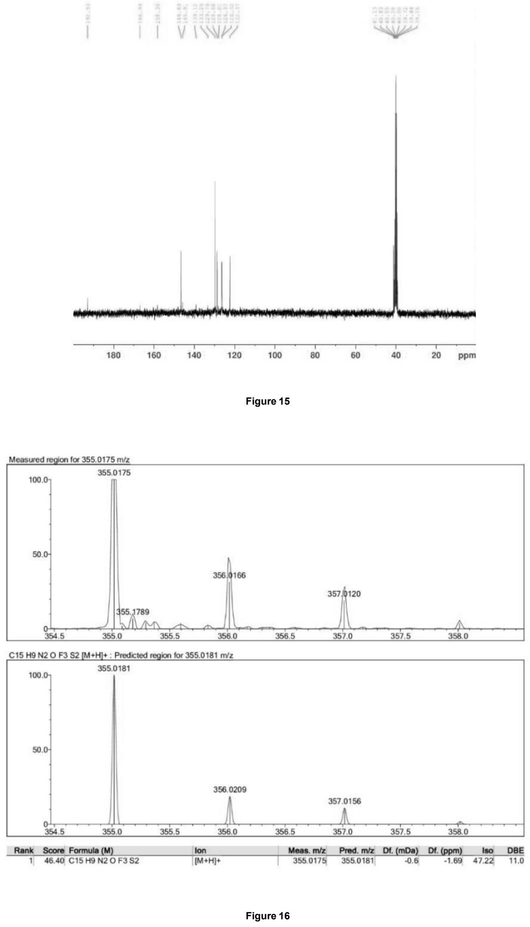

The characteristic properties of Compound 4 are given below.
- The melting point is 107-109°C.
- IR spectrum, IR vₘₐₓ (cm⁻¹): 3061.03, 3049.46 (aromatic C-H stretching bands), 2960.73, 2922.16 (aliphatic C-H stretching bands), 1681.93 (C=O stretching band), 1577.77, 1552.70, 1512.19 (C=C and C=N stretching bands), 1433.11, 1413.82, 1377.17, 1325.10, 1313.52, 1286.52, 1220.94, 1192.01, 1165.00, 1124.50, 1105.21, 1064.71, 1010.70 (C-H bending, C-N stretching and aromatic C-H in-plane bending bands), 997.20, 964.41, 829.39, 806.25, 742.59, 713.66, 678.94, 655.80, 597.93 (aromatic C-H out-of-plane bending bands and C-S stretching band) (Figure 13).
- ¹H-NMR spectrum, ¹H-NMR (300 MHz, DMSO-d₆) δ (ppm): 5.26 (s, 2H), 7.50 (dd, *J* = 4.71, 8.22 Hz, 1H), 7.97 (d, *J* = 8.25 Hz, 2H), 8.12 (dd, *J* = 1.53, 8.22 Hz, 1H), 8.28 (d, *J* = 8.10 Hz, 2H), 8.49 (dd, *J* = 1.53, 4.71 Hz, 1H) (Figure 14).
- ¹³C-NMR spectrum, ¹³C-NMR (75 MHz, DMSO-d₆) δ (ppm): 41.13 (CH₂), 122.37 (CH), 126.35 (d, *J* = 3.54 Hz, 2CH), 128.01 (C), 128.68 (CH), 129.76 (2CH), 133.25 (C), 139.12 (C), 145.91 (C), 146.63 (CH), 158.30 (C), 166.99 (C), 192.93 (C) (Figure 15).
- HRMS spectrum, HRMS (ESI) *(m*/*z):* [M+H]⁺ calculated for C₁₅H₉F₃N₂OS₂: 355.0181, found: 355.0175 (Figure 16).

The synthesis method of Compound 4 comprises the process steps of;
**a.** adding thiazolo[5,4-*b*]pyridin-2(1H)-thione, 2-bromo-4'-trifluoromethylacetophenone and a potassium carbonate catalyst in equivalent amounts,
**b.** adding 20 mL of acetone to the mixture,
**c.** completing the reaction by stirring the acetone-added mixture at room temperature for 6 hours,
**d.** subsequently evaporating acetone, which is the solvent of the reaction medium, and washing the resulting solid product with water, and
**e.** drying the washed solid product and crystallising it from ethanol.

The compound 1-(4-Cyanophenyl)-2-(thiazolo[5,4-*b*]pyridin-2-ylthio)ethan-1-one (Compound 5), obtained when the R group in said Formula X is R=R₅, is represented by Formula 5.

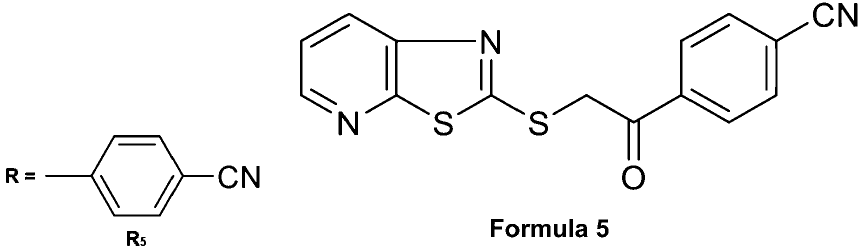

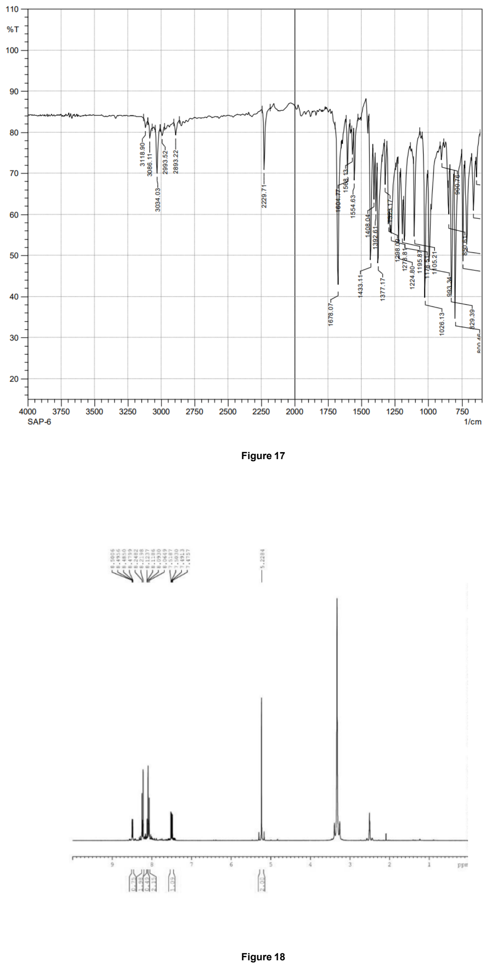

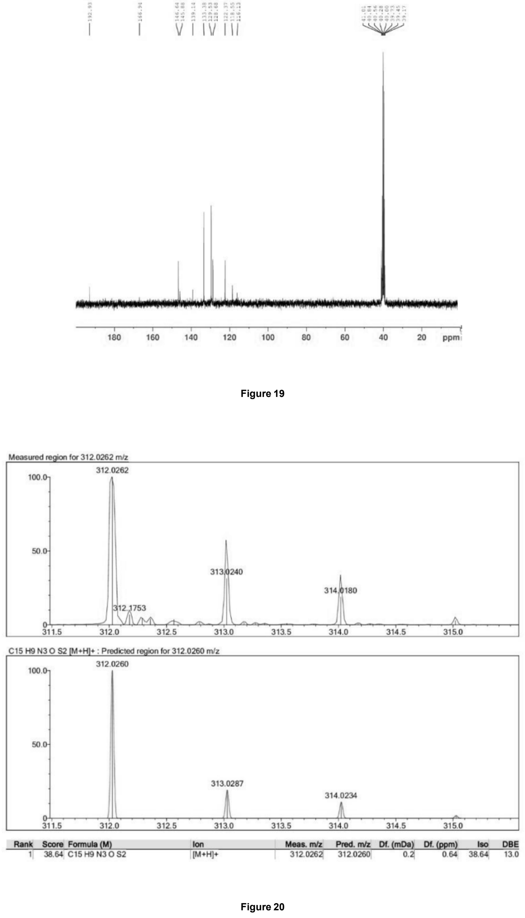

The characteristic properties of Compound 5 are given below.
- The melting point is 127-129°C.
- IR spectrum, IR vₘₐₓ (cm⁻¹): 3118.90, 3086.11, 3034.03 (aromatic C-H stretching bands), 2993.52, 2893.22 (aliphatic C-H stretching bands), 2229.71 (C≡N stretching band), 1678.07 (C=O stretching band), 1604.77, 1568.13, 1554.63 (C=C and C=N stretching bands), 1433.11, 1408.04, 1392.61, 1377.17, 1323.17, 1298.09, 1278.81, 1224.80, 1195.87, 1178.51, 1105.21, 1026.13 (C-H bending, C-N, C-O stretching and aromatic C-H in-plane bending bands), 993.34, 900.76, 850.61, 829.39, 800.46, 742.59, 711.73, 663.51, 640.37 (aromatic C-H out-of-plane bending bands and C-S stretching band) (Figure 17).
- ¹H-NMR spectrum, ¹H-NMR (300 MHz, DMSO-d₆) δ (ppm): 5.23 (s, 2H), 7.50 (dd, *J* = 4.71, 8.22 Hz, 1H), 8.08 (d, *J* = 8.43 Hz, 2H), 8.12 (d, *J* = 1.53 Hz, 1H), 8.23 (d, *J* = 8.52 Hz, 2H), 8.49 (dd, *J* = 1.50, 4.68 Hz, 1H) (Figure 18).
- ¹³C-NMR spectrum, ¹³C-NMR (75 MHz, DMSO-d₆) δ (ppm): 41.01 (CH₂), 116.13 (C), 118.55 (C), 122.37 (CH), 124.29 (C), 128.68 (CH), 129.53 (2CH), 133.38 (2CH), 139.14 (C), 145.88 (C), 146.64 (CH), 166.94 (C), 192.93 (C) (Figure 19).
- HRMS spectrum, HRMS (ESI) *(m*/*z):* [M+H]⁺ calculated for C₁₅H₉N₃OS₂: 312.0260, found: 312.0262 (Figure 20).

The synthesis method of Compound 5 comprises the process steps of;
**a.** adding thiazolo[5,4-*b*]pyridin-2(1*H*)-thione, 2-bromo-4'-cyanoacetophenone and a potassium carbonate catalyst in equivalent amounts,
**b.** adding 20 mL of acetone to the mixture,
**c.** completing the reaction by stirring the acetone-added mixture at room temperature for 6 hours,
**d.** subsequently evaporating acetone, which is the solvent of the reaction medium, and washing the resulting solid product with water, and
**e.** drying the washed solid product and crystallising it from ethanol.

The compound 1-(4-Methylphenyl)-2-(thiazolo[5,4-*b*]pyridin-2-ylthio)ethan-1-one (Compound 6), obtained when the R group in said Formula X is R=R₆, is represented by Formula 6.

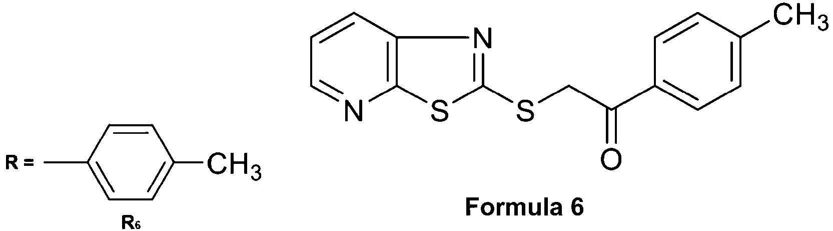

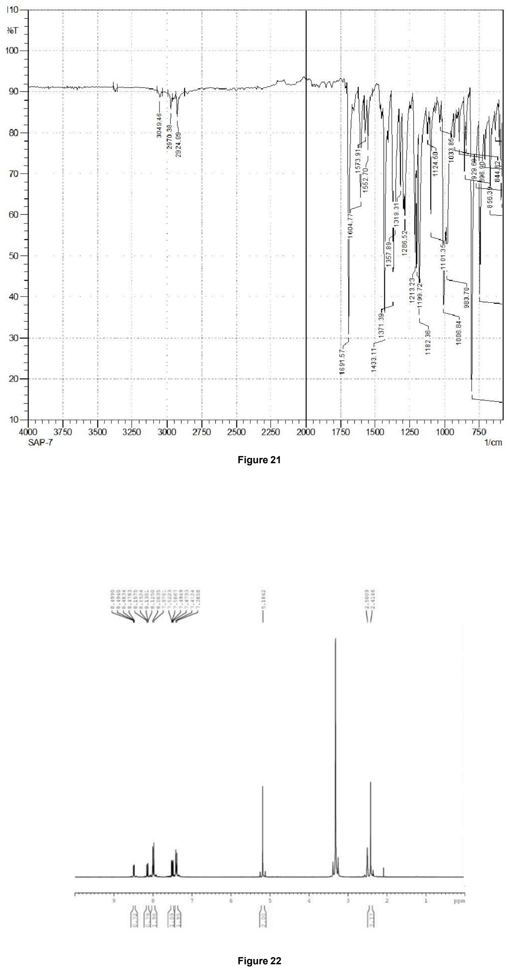

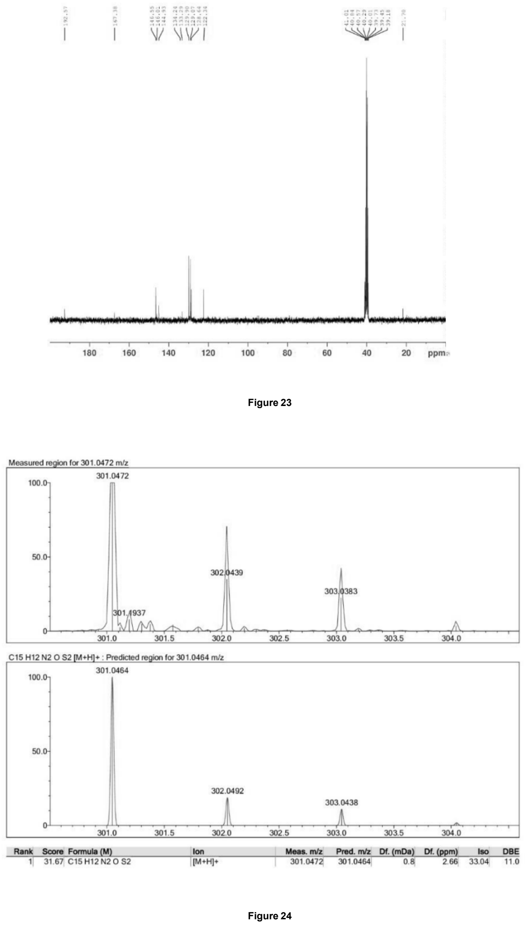

The characteristic properties of Compound 6 are given below.
- The melting point is 118-119°C.
- IR spectrum, IR vₘₐₓ (cm⁻¹): 3049.46 (aromatic C-H stretching band), 2970.38, 2924.09 (aliphatic C-H stretching bands), 1691.57 (C=O stretching band), 1604.77, 1573.91, 1552.70 (C=C and C=N stretching bands), 1433.11, 1371.39, 1357.89, 1319.31, 1286.52, 1213.23, 1199.72, 1182.36, 1124.50, 1101.35, 1033.85, 1006.84 (C-H bending, C-N stretching and aromatic C-H in-plane bending bands), 983.70, 929.69, 896.90, 856.39, 844.82, 804.32, 777.31, 746.45, 709.80, 671.23, 636.51, 607.58, 590.22 (aromatic C-H out-of-plane bending bands and C-S stretching band) (Figure 21).
- ¹H-NMR spectrum, ¹H-NMR (300 MHz, DMSO-d₆) δ (ppm): 2.41 (s, 3H), 5.18 (s, 2H), 7.40 (d, *J* = 7.98 Hz, 2H), 7.50 (dd, *J* = 4.68, 8.22 Hz, 1H), 7.99 (d, J = 8.22 Hz, 2H), 8.14 (dd, *J* = 1.53, 8.22 Hz, 1H), 8.49 (dd, *J* = 1.50, 4.68 Hz, 1H) (Figure 22).
- ¹³C-NMR spectrum, ¹³C-NMR (75 MHz, DMSO-d₆) δ (ppm): 21.70 (CH₃), 41.01 (CH₂), 122.34 (CH), 128.64 (CH), 129.07 (2CH), 129.90 (2CH), 133.29 (C), 134.24 (C), 144.93 (C), 146.01 (C), 146.55 (CH), 167.38 (C), 192.57 (C) (Figure 23).
- HRMS spectrum, HRMS (ESI) *(m*/*z):* [M+H]⁺ calculated for C₁₅H₁₂N₂OS₂: 301.0464, found: 301.0472 (Figure 24).

The synthesis method of Compound 6 comprises the process steps of;
**a.** adding thiazolo[5,4-*b*]pyridin-2(1*H*)-thione, 2-bromo-4'-methylacetophenone and a potassium carbonate catalyst in equivalent amounts,
**b.** adding 20 mL of acetone to the mixture,
**c.** completing the reaction by stirring the acetone-added mixture at room temperature for 6 hours,
**d.** subsequently evaporating acetone, which is the solvent of the reaction medium, and washing the resulting solid product with water, and
**e.** drying the washed solid product and crystallising it from ethanol.

The compound 1-(4-Methoxyphenyl)-2-(thiazolo[5,4-*b*]pyridin-2-ylthio)ethan-1-one (Compound 7), obtained when the R group in said Formula X is R=R₇, is represented by Formula 7.

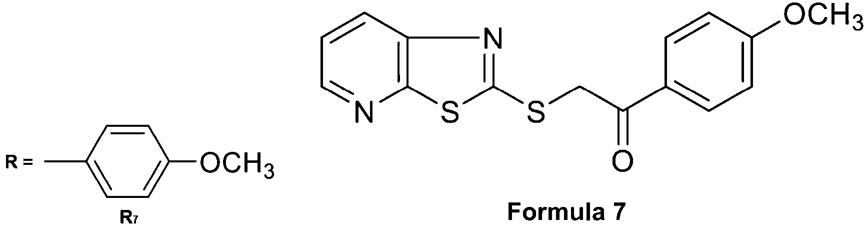

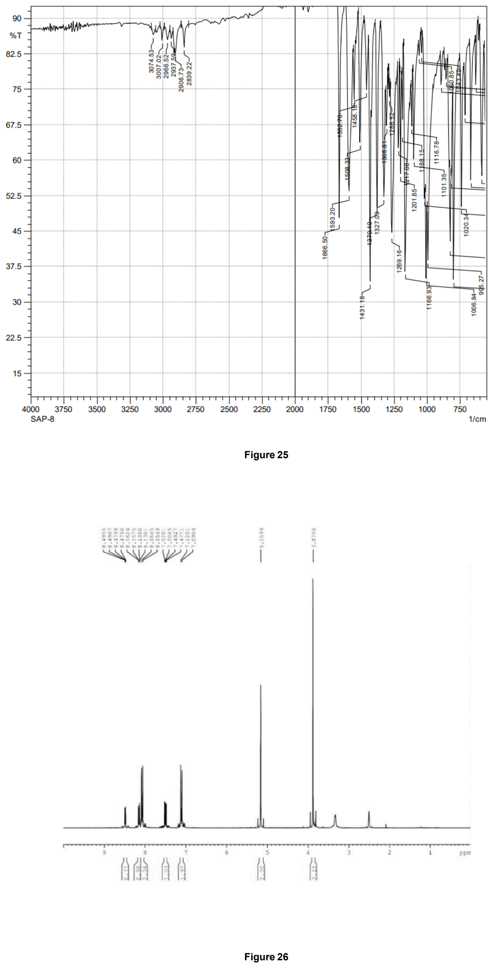

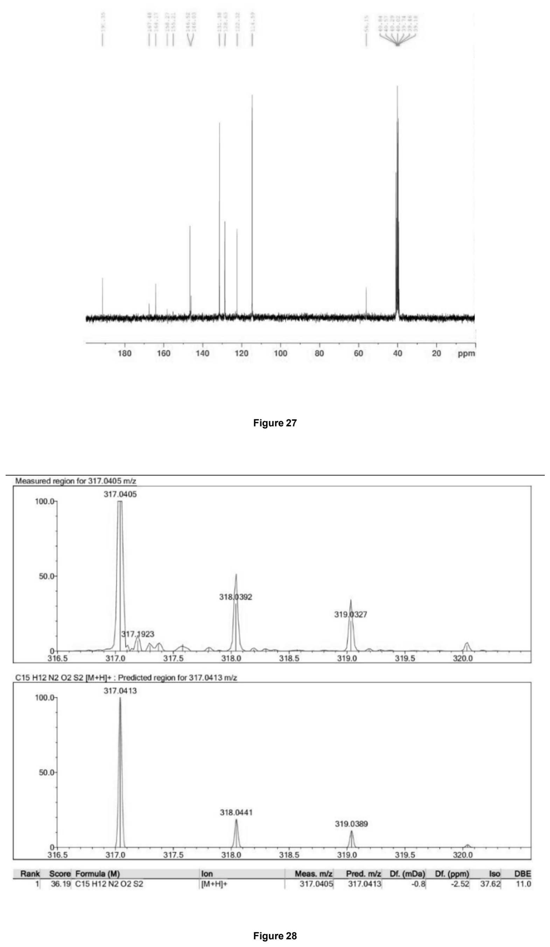

The characteristic properties of Compound 7 are given below.
- The melting point is 122-124°C.
- IR spectrum, IR vₘₐₓ (cm⁻¹): 3074.53, 3007.02 (aromatic C-H stretching bands), 2966.52, 2937.59, 2906.73, 2839.22 (aliphatic C-H stretching bands), 1666.50 (C=O stretching band), 1593.20, 1552.70, 1508.33, 1458.18 (C=C and C=N stretching bands), 1431.18, 1379.10, 1327.03, 1305.81, 1286.52, 1269.16, 1217.08, 1201.65, 1188.15, 1166.93, 1116.78, 1101.35, 1060.85, 1043.49, 1020.34, 1006.84 (C-H bending, C-N, C-O stretching and aromatic C-H in-plane bending bands), 995.27, 893.04, 856.39, 827.46, 817.82, 800.46, 740.67, 713.66, 669.30, 630.72, 588.29, 561.29 (aromatic C-H out-of-plane bending bands and C-S stretching band) (Figure 25).
- ¹H-NMR spectrum, ¹H-NMR (300 MHz, DMSO-d₆) δ (ppm): 3.88 (s, 3H), 5.16 (s, 2H), 7.11 (d, *J* = 8.91 Hz, 2H), 7.50 (dd, *J* = 4.68, 8.22 Hz, 1H), 8.07 (d, *J* = 8.88 Hz, 2H), 8.15 (dd, *J* = 1.47, 8.22 Hz, 1H), 8.49 (dd, *J* = 1.44, 4.68 Hz, 1H) (Figure 26).
- ¹³C-NMR spectrum, ¹³C-NMR (75 MHz, DMSO-d₆) δ (ppm): 40.84 (CH₂), 56.15 (CH₃), 114.59 (2CH), 122.32 (CH), 128.63 (CH), 131.38 (2CH), 146.03 (C), 146.52 (CH), 155.21 (C), 158.27 (C), 164.17 (C), 167.48 (C), 191.35 (C) (Figure 27).
- HRMS spectrum, HRMS (ESI) *(m*/*z):* [M+H]⁺ calculated for C₁₅H₁₂N₂O₂S₂: 317.0413, found: 317.0405 (Figure 28).

The synthesis method of Compound 7 comprises the process steps of;
**a.** adding thiazolo[5,4-*b*]pyridin-2(1H)-thione, 2-bromo-4'-methoxyacetophenone and a potassium carbonate catalyst in equivalent amounts,
**b.** adding 20 mL of acetone to the mixture,
**c.** completing the reaction by stirring the acetone-added mixture at room temperature for 6 hours,
**d.** subsequently evaporating acetone, which is the solvent of the reaction medium, and washing the resulting solid product with water, and
**e.** drying the washed solid product and crystallising it from ethanol.

The compound 1-(4-Nitrophenyl)-2-(thiazolo[5,4-*b*]pyridin-2-ylthio)ethan-1-one (Compound 8), obtained when the R group in said Formula X is R=R₈, is represented by Formula 8.

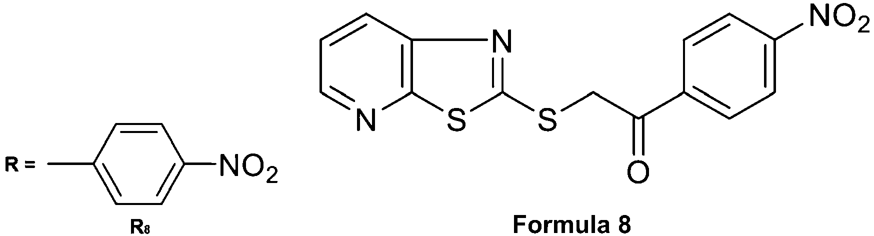

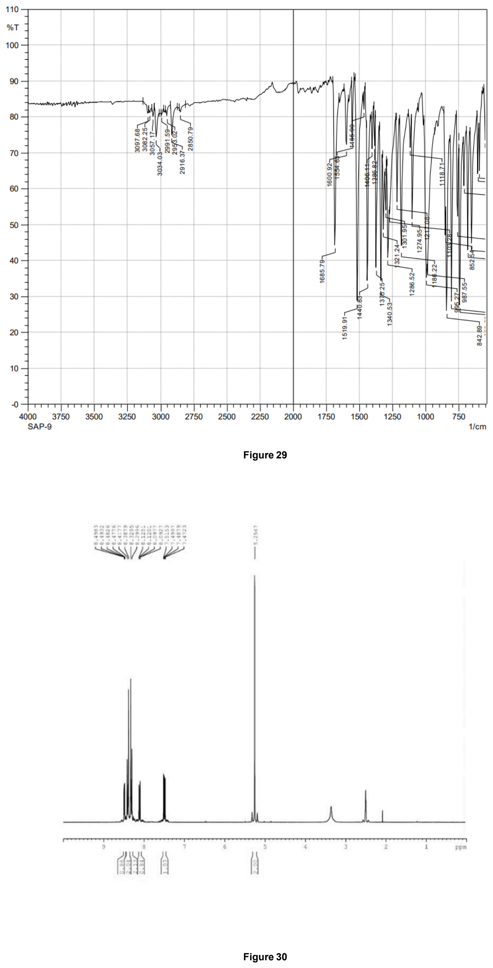

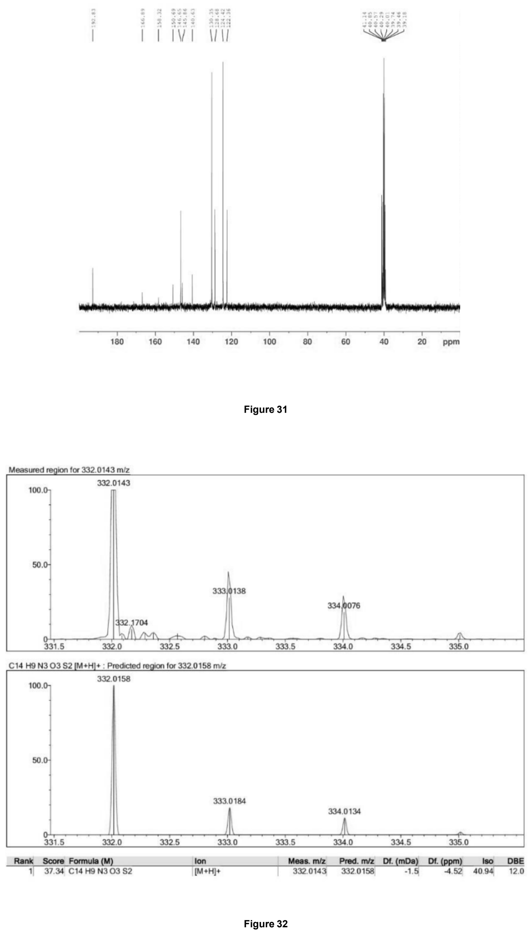

The characteristic properties of Compound 8 are given below.
- The melting point is 169-171°C.
- IR spectrum, IR vₘₐₓ (cm⁻¹): 3097.68, 3082.25, 3057.17, 3034.03 (aromatic C-H stretching bands), 2991.59, 2953.02, 2916.37, 2850.79 (aliphatic C-H stretching bands), 1685.79 (C=O stretching band), 1600.92, 1554.63, 1519.91, 1465.90 (NO₂, C=C and C=N stretching bands), 1440.83, 1406.11, 1386.82, 1375.25, 1340.53, 1321.24, 1301.95, 1286.52, 1274.95, 1217.08, 1186.22, 1118.71, 1103.28 (C-H bending, NO₂, C-N stretching and aromatic C-H in-plane bending bands), 995.27, 987.55, 852.54, 842.89, 806.25, 761.88, 744.52, 711.73, 682.80, 655.80, 607.58, 596.00, 553.57 (aromatic C-H out-of-plane bending bands and C-S stretching band) (Figure 29).
- ¹H-NMR spectrum, ¹H-NMR (300 MHz, DMSO-d₆) δ (ppm): 5.25 (s, 2H), 7.50 (dd, *J* = 4.68, 8.22 Hz, 1H), 8.11 (dd, *J* = 1.50, 8.22 Hz, 1H), 8.31 (d, *J* = 8.97 Hz, 2H), 8.40 (d, *J* = 8.94 Hz, 2H), 8.49 (dd, *J* = 1.53, 4.71 Hz, 1H) (Figure 30).
- ¹³C-NMR spectrum, ¹³C-NMR (75 MHz, DMSO-d₆) δ (ppm): 41.14 (CH₂), 122.36 (CH), 124.42 (2CH), 128.68 (CH), 130.35 (2CH), 140.63 (C), 145.86 (C), 146.65 (CH), 150.69 (C), 158.32 (C), 166.89 (C), 192.83 (C) (Figure 31).
- HRMS spectrum, HRMS (ESI) *(m*/*z):* [M+H]⁺ calculated for C₁₄H₉N₃O₃S₂: 332.0158, found: 332.0143 (Figure 32).

The synthesis method of Compound 8 comprises the process steps of;
**a.** adding thiazolo[5,4-*b*]pyridin-2(1*H*)-thione, 2-bromo-4'-nitroacetophenone and a potassium carbonate catalyst in equivalent amounts,
**b.** adding 20 mL of acetone to the mixture,
**c.** completing the reaction by stirring the acetone-added mixture at room temperature for 6 hours,
**d.** subsequently evaporating acetone, which is the solvent of the reaction medium, and washing the resulting solid product with water, and
**e.** drying the washed solid product and crystallising it from ethanol.

The compound 1-(4-Methylsulfonylphenyl)-2-(thiazolo[5,4-*b*]pyridin-2-ylthio)ethan-1-one (Compound 9), obtained when the R group in said Formula X is R=R₉, is represented by Formula 9.

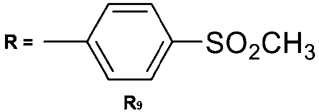

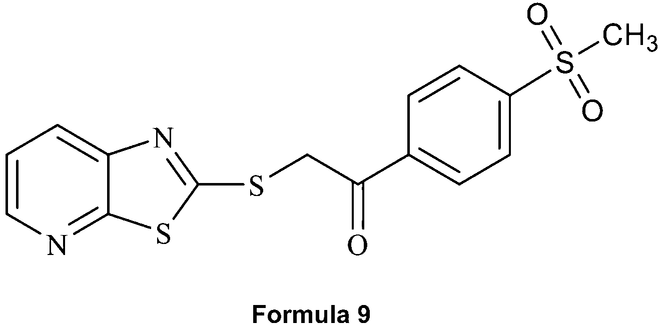

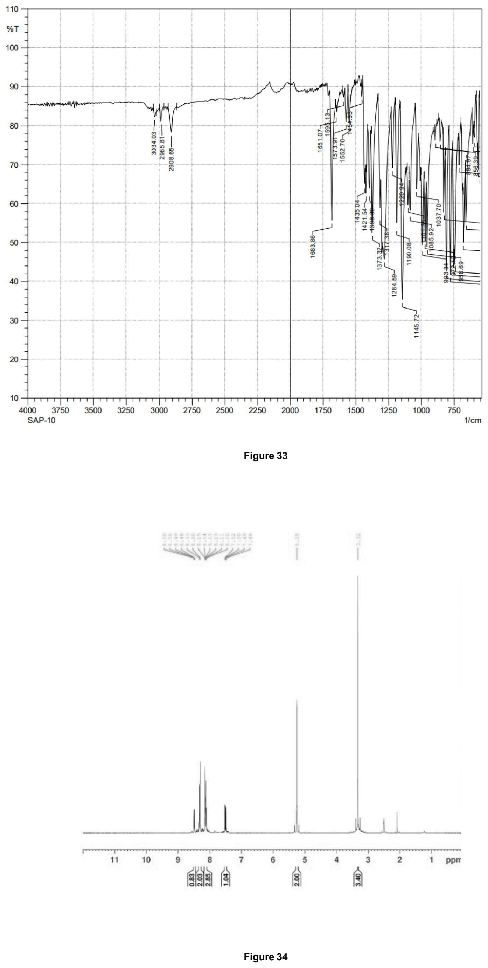

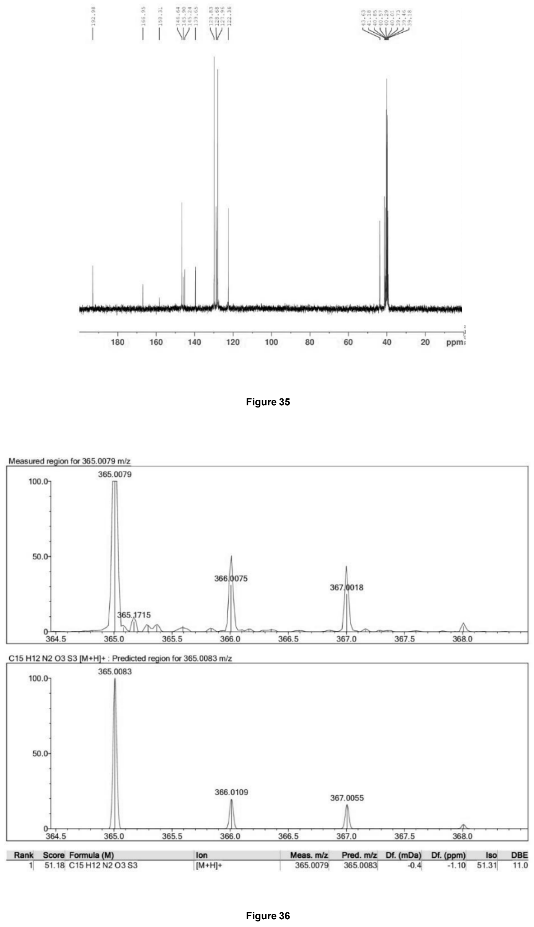

The characteristic properties of Compound 9 are given below.
- The melting point is 149-151°C.
- IR spectrum, IR vₘₐₓ (cm⁻¹): 3034.03 (aromatic C-H stretching band), 2985.81, 2908.65 (aliphatic C-H stretching bands), 1683.86 (C=O stretching band), 1651.07, 1595.13, 1573.91, 1552.70, 1454.33 (C=C and C=N stretching bands), 1435.04, 1421.54, 1398.39, 1373.32, 1317.38, 1300.02, 1284.59, 1220.94, 1190.08, 1145.72, 1101.35, 1085.92, 1037.70, 1010.70 (SO₂ stretching, C-H bending, C-N stretching and aromatic C-H in-plane bending bands), 993.34, 972.12, 956.69, 894.97, 856.39, 829.39, 808.17, 779.24, 752.24, 744.52, 711.73, 678.94, 657.73, 607.58, 597.93, 567.07, 553.57 (aromatic C-H out-of-plane bending bands and C-S stretching band) (Figure 33).
- ¹H-NMR spectrum, ¹H-NMR (300 MHz, DMSO-d₆) δ (ppm): 3.32 (s, 3H), 5.25 (s, 2H), 7.50 (dd, *J* = 4.71, 8.22 Hz, 1H), 8.11 (d, *J* = 1.53 Hz, 2H), 8.14 (dd, *J* = 5.19, 6.69 Hz, 1H), 8.31 (d, *J* = 8.52 Hz, 2H), 8.49 (dd, *J* = 1.53, 4.71 Hz, 1H) (Figure 34).
- ¹³C-NMR spectrum, ¹³C-NMR (75 MHz, DMSO-d₆) δ (ppm): 41.18 (CH₂), 43.63 (CH₃), 122.36 (CH), 127.96 (2CH), 128.68 (CH), 129.83 (2CH), 139.65 (C), 145.24 (C), 145.90 (C), 146.64 (CH), 158.31 (C), 166.95 (C), 192.98 (C) (Figure 35).
- HRMS spectrum, HRMS (ESI) *(m*/*z):* [M+H]⁺ calculated for C₁₅H₁₂N₂O₃S₃: 365.0083, found: 365.0079 (Figure 36).

The synthesis method of Compound 9 comprises the process steps of;
**a.** adding thiazolo[5,4-*b*]pyridin-2(1H)-thione, 2-bromo-4'-methylsulfonylacetophenone and a potassium carbonate catalyst in equivalent amounts,
**b.** adding 20 mL of acetone to the mixture,
**c.** completing the reaction by stirring the acetone-added mixture at room temperature for 6 hours,
**d.** subsequently evaporating acetone, which is the solvent of the reaction medium, and washing the resulting solid product with water, and
**e.** drying the washed solid product and crystallising it from ethanol.

The compound 1-(Naphthalen-2-yl)-2-(thiazolo[5,4-*b*]pyridin-2-ylthio)ethan-1-one (Compound 10), obtained when the R group in said Formula X is R=R₁₀, is represented by Formula 10.

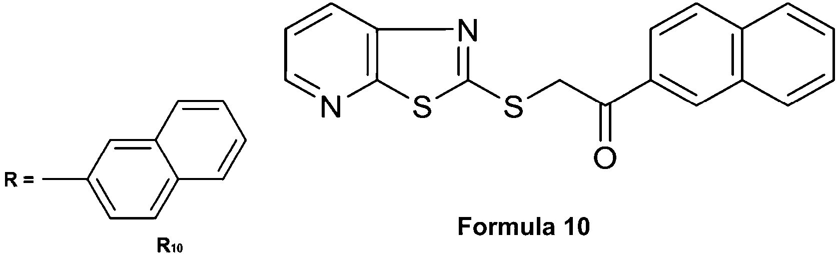

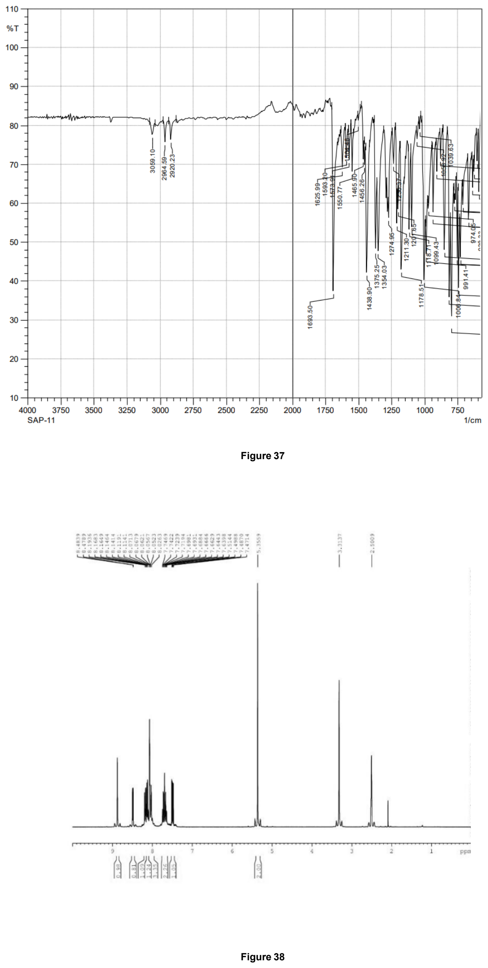

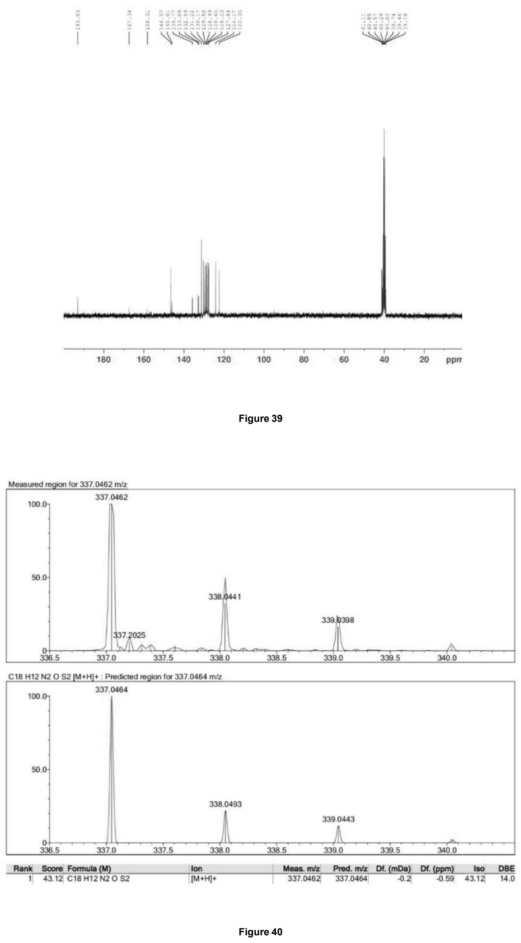

The characteristic properties of Compound 10 are given below.
- The melting point is 167-169°C.
- IR spectrum, IR vₘₐₓ (cm⁻¹): 3059.10 (aromatic C-H stretching band), 2964.59, 2920.23 (aliphatic C-H stretching bands), 1693.50 (C=O stretching band), 1625.99, 1593.20, 1573.91, 1550.77, 1504.48, 1465.90 (C=C and C=N stretching bands), 1438.90, 1375.25, 1354.03, 1274.95, 1236.37, 1211.30, 1201.65, 1178.51, 1118.71, 1099.43, 1058.92, 1039.63, 1006.84 (C-H bending, C-N stretching and aromatic C-H in-plane bending bands), 991.41, 974.05, 939.33, 910.40, 881.47, 850.61, 815.89, 800.46, 775.38, 756.10, 742.59, 731.02, 711.73, 669.30, 638.44, 590.22 (aromatic C-H out-of-plane bending bands and C-S stretching band) (Figure 37).
- ¹H-NMR spectrum, ¹H-NMR (300 MHz, DMSO-d₆) δ (ppm): 5.36 (s, 2H), 7.50 (dd, *J* = 4.68, 8.22 Hz, 1H), 7.64-7.75 (m, 2H), 8.03 (br s, 1H), 8.06 (m, 2H), 8.13 (dd, *J* = 1.50, 8.19 Hz, 1H), 8.18 (d, *J* = 7.59 Hz, 1H), 8.49 (dd, *J* = 1.47, 4.65 Hz, 1H), 8.88 (s, 1H) (Figure 38).
- ¹³C-NMR spectrum, ¹³C-NMR (75 MHz, DMSO-d₆) δ (ppm): 41.11 (CH₂), 122.35 (CH), 124.17 (CH), 127.64 (CH), 128.23 (CH), 128.65 (CH), 128.99 (CH), 129.50 (CH), 130.17 (CH), 131.22 (CH), 132.59 (C), 133.09 (C), 135.77 (C), 146.01 (C), 146.57 (CH), 158.31 (C), 167.34 (C), 193.03 (C) (Figure 39).
- HRMS spectrum, HRMS (ESI) *(m*/*z):* [M+H]⁺ calculated for C₁₈H₁₂N₂OS₂: 337.0464, found: 337.0462 (Figure 40).

The synthesis method of Compound 10 comprises the process steps of;
**a.** adding thiazolo[5,4-*b*]pyridin-2(1*H*)-thione, 2-bromo-2'-acetonaphthone and a potassium carbonate catalyst in equivalent amounts,
**b.** adding 20 mL of acetone to the mixture,
**c.** completing the reaction by stirring the acetone-added mixture at room temperature for 6 hours,
**d.** subsequently evaporating acetone, which is the solvent of the reaction medium, and washing the resulting solid product with water, and
**e.** drying the washed solid product and crystallising it from ethanol.

The compound 1-(1,3-Benzodioxol-5-yl)-2-(thiazolo[5,4-*b*]pyridin-2-ylthio)ethan-1-one (Compound 11), obtained when the R group in said Formula X is R=R₁₁, is represented by Formula 11.

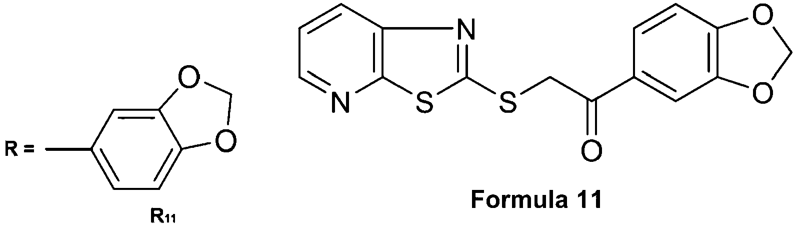

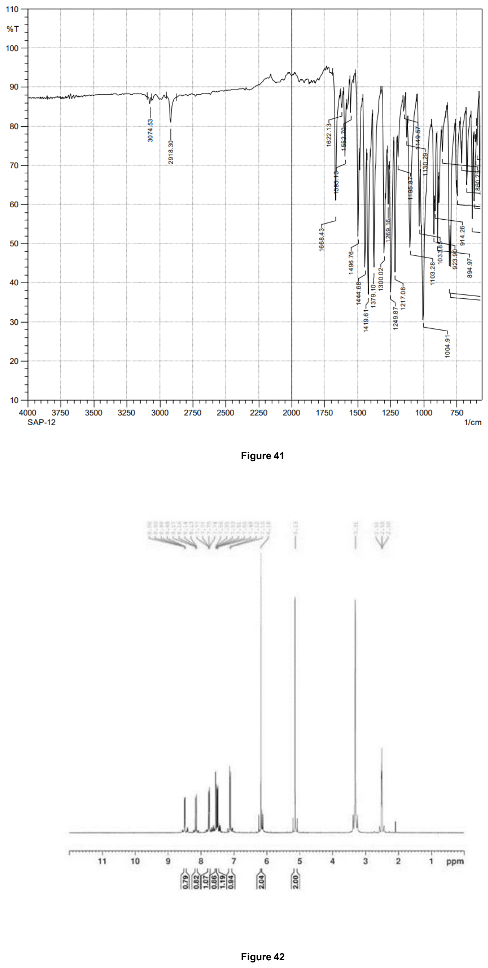

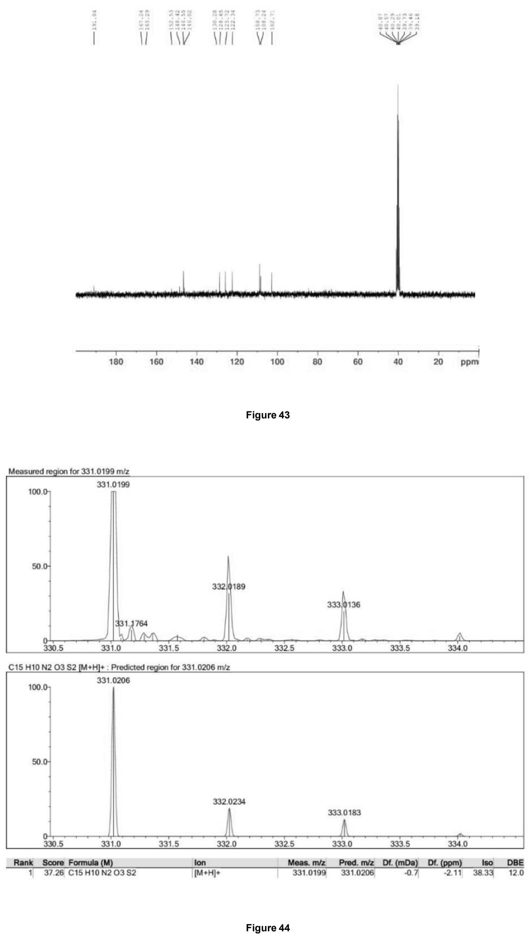

The characteristic properties of Compound 11 are given below.
- The melting point is 146-148°C.
- IR spectrum, IR vₘₐₓ (cm⁻¹): 3074.53 (aromatic C-H stretching band), 2918.30 (aliphatic C-H stretching band), 1668.43 (C=O stretching band), 1622.13, 1595.13, 1552.70, 1496.76 (C=C and C=N stretching bands), 1444.68, 1419.61, 1379.10, 1300.02, 1269.16, 1249.87, 1217.08, 1149.57, 1130.29, 1103.28, 1033.85, 1004.91 (C-H bending, C-N, C-O stretching and aromatic C-H in-plane bending bands), 923.90, 914.26, 894.97, 860.25, 808.17, 798.53, 748.38, 719.45, 675.09, 634.58, 617.22, 607.58, 597.93, 574.79 (aromatic C-H out-of-plane bending bands and C-S stretching band) (Figure 41).
- ¹H-NMR spectrum, ¹H-NMR (300 MHz, DMSO-d₆) δ (ppm): 5.13 (s, 2H), 6.18 (s, 2H), 7.11 (d, *J* = 8.19 Hz, 1H), 7.50 (dd, *J* = 4.71, 8.22 Hz, 1H), 7.55 (d, *J* = 1.71 Hz, 1H), 7.76 (dd, *J* = 1.77, 8.19 Hz, 1H), 8.15 (dd, *J* = 1.50, 8.22 Hz, 1H), 8.49 (dd, J = 1.50, 4.68 Hz, 1H) (Figure 42).
- ¹³C-NMR spectrum, ¹³C-NMR (75 MHz, DMSO-d₆) δ (ppm): 40.87 (CH₂), 102.71 (CH₂), 108.24 (CH), 108.73 (CH), 122.34 (CH), 125.72 (CH), 128.65 (CH), 130.28 (C), 146.02 (C), 146.55 (CH), 148.42 (C), 152.54 (C), 165.43 (C), 167.42 (C), 191.04 (C) (Figure 43).
- HRMS spectrum, HRMS (ESI) *(m*/*z):* [M+H]⁺ calculated for C₁₅H₁₀N₂O₃S₂: 331.0206, found: 331.0199 (Figure 44).

The synthesis method of Compound 11 comprises the process steps of;
**a.** adding thiazolo[5,4-*b*]pyridin-2(1*H*)-thione, 1-(1,3-benzodioxol-5-yl)-2-bromoethanone and a potassium carbonate catalyst in equivalent amounts,
**b.** adding 20 mL of acetone to the mixture,
**c.** completing the reaction by stirring the acetone-added mixture at room temperature for 6 hours,
**d.** subsequently evaporating acetone, which is the solvent of the reaction medium, and washing the resulting solid product with water, and
**e.** drying the washed solid product and crystallising it from ethanol.

During the anticancer activity studies, the *in vitro* cytotoxic effects of the compounds were determined by the MTT method. For this purpose, firstly, the cytotoxic effects of the compounds on the MCF-7 human breast adenocarcinoma cell line were investigated. Subsequently, their cytotoxicities on the NIH/3T3 mouse embryonic fibroblast (healthy) cell line were evaluated, and IC₅₀ values for the NIH/3T3 cell line and the MCF-7 cell line, as well as selectivity index values (SI, IC₅₀ for the NIH/3T3 cell line / IC₅₀ for the MCF-7 cell line), were determined. Fetal calf serum, 100 IU/mL penicillin and 100 mg/mL streptomycin were added to the culture medium. RPMI 1640 was used as the culture medium for MCF-7 cells, and Dulbecco's Modified Eagle Medium (DMEM) was used for NIH/3T3 cells. As positive controls, doxorubicin, which is routinely used in cancer treatment today, and letrozole, which is an aromatase enzyme inhibitor, were used.

For the proliferation and preparation of the MCF-7 and NIH/3T3 cell lines used in the experiments, routine passaging was performed every 2-3 days. The cell flask removed from the incubator was gently shaken to allow dead cells to pass into the culture medium solution, and then the culture medium inside the cell flask was removed and discarded using a sterile pipette. For washing the cells, 5 mL of phosphate buffer was added to the culture flask and the cells were washed, and the washing solution was removed from the environment. Trypsin-EDTA solution (1X) (1-3 mL for 75 cm² culture flasks, 0.5-1.5 mL for 25 cm² culture flasks) was added to the culture flask, gently shaken, and then kept in the incubator for approximately 5 minutes (%5 CO₂, %95 humidity and 37 °C). The cell flask removed from the incubator was supplemented with 20-25 mL of culture medium to suspend the cells, and the cells were split at ratios of 1:2 and 1:3 and transferred into new cell flasks. The cell flasks were placed in the incubator and left for incubation (5% CO₂, 95% humidity and 37 °C).

The cell culture flask removed from the incubator was gently shaken to allow dead cells to pass into the culture medium solution, and then the culture medium inside the flask was removed and discarded using a sterile pipette. Trypsin-EDTA solution (1X) (1-3 mL for 75 cm² culture flasks, 0.5-1.5 mL for 25 cm² culture flasks) was added to the culture flask, gently shaken, and then kept in the incubator for approximately 5 minutes (5% CO₂, 95% humidity and 37 °C). Culture medium (at least twice the volume of the added trypsin-EDTA solution) was added into the culture flasks removed from the incubator, and the contents were transferred into a centrifuge tube using a pipette. After shaking the cell suspension in the centrifuge tube, 10 µL was taken and counted using an automatic cell counting device. The cell suspension was transferred into cuvettes and distributed into a 96-well cell culture plate at 200 µL/well (1 × 10⁴ cells/100 µL), and incubated for 24 hours (5% CO₂, 95% humidity and 37 °C).

At the end of the 24-hour incubation period, the plates were inverted and the culture medium was removed. Ten serial concentrations of the compounds in the range of 0.0000316 mM-1 mM (stock solutions prepared in dimethyl sulfoxide (DMSO)) were applied to the plates together with the positive controls. The plates were incubated for 24 hours (5% CO₂, 95% humidity and 37 °C). After the 24-hour incubation period, the upper part of the cell cultures was removed by inverting the plates. The cells were washed with phosphate buffer and the washing solution was removed from the environment. The MTT solution (5 mg/mL) and the culture medium were mixed at a ratio of 1:10. This solution mixture was added to the cell culture plate at 100 µL/well. The plates were incubated for 3 hours (5% CO₂, 95% humidity and 37 °C). At the end of the 3-hour incubation period, the plates were inverted and the upper part was discarded. DMSO solution was added to the cell culture plate at 100 µL/well, and the optical density values of the wells were read at 540 nm using ELISA. Percentage inhibition values were calculated for each concentration of the compounds. The inhibitory concentration 50 (IC₅₀) values of the substances were calculated by nonlinear regression analysis, and the cytotoxic properties of the substances were interpreted [1-3].

The aromatase inhibitor activities of the compounds were investigated using the "Aromatase (CYP19A) Inhibitor Screening Kit (fluorometric)". Letrozole, which is an aromatase enzyme inhibitor, was used as a reference substance. The inhibitory activity is based on the fluorometric measurement of the substrate and the enzyme. In the presence of an inhibitor specific to the aromatase enzyme, the enzyme loses its activity and the absorbance decreases. The biological activities of the compounds were primarily tested by preparing them at two concentrations of 10⁻³ and 10⁻⁴ M in 2% DMSO. Activity values were evaluated as inhibition percentages in the range of 0-100% [4-6].

The IC₅₀ values, SI values and IC₅₀ values related to aromatase inhibition determined in the invention for the NIH/3T3 and MCF-7 cell lines are shown in Table 1. The test results are expressed as the averages of quadruplicate experiments.

Compared to doxorubicin (IC₅₀ = 1.940±0.084 µM), which is used in breast cancer treatment; 2-(thiazolo[5,4-*b*]pyridin-2-ylthio)-1-(4-nitrophenyl)ethan-1-one (Compound 8), 1-(4-cyanophenyl)-2-(thiazolo[5,4-*b*]pyridin-2-ylthio)ethan-1-one (Compound 5), 1-(4-chlorophenyl)-2-(thiazolo[5,4-*b*]pyridin-2-ylthio)ethan-1-one (Compound 1), and 1-(4-methoxyphenyl)-2-(thiazolo[5,4-*b*]pyridin-2-ylthio)ethan-1-one (Compound 7) were found to have strong anticancer activity on the MCF-7 cell line with IC₅₀ values of 2.120±0.836, 3.234±0.149, 3.741±0.174 and 4.926±0.213 µM, respectively. The SI values for Compound 8, Compound 5, Compound 1 and Compound 7 were found to be 15.759, 13.965, 12.106 and 10.677, respectively. Considering the SI values, it was determined that the compounds coded as Compound 8, Compound 5, Compound 1 and Compound 7 exhibited selective anticancer activity on the MCF-7 cell line. In addition, when compared to letrozole, which is an aromatase inhibitor (IC₅₀ = 0.024±0.001 µg/mL), Compound 8, Compound 1, Compound 5 and Compound 7 exhibited very strong aromatase inhibitory activity at the micromolar level with IC₅₀ values of 0.029±0.001 µg/mL, 0.034±0.001 µg/mL, 0.045±0.020 µg/mL and 0.057±0.002 µg/mL, respectively.

**Table 1. IC₅₀ values related to the cytotoxicities of the compounds on the MCF-7 and NIH/3T3 cell lines and their aromatase inhibitor effects**

| **Compound** | **IC₅₀ (µM)** | | **SI** | **Aromatase Inhibition (IC₅₀, µM)** |
|---|---|---|---|---|
| | **MCF-7** | **NIH/3T3** | | |
| **1** | 3.741±0.174 | 45.289±1.962 | 12.106 | 0.034±0.001 |
| **2** | 13.956±0.512 | 21.980±0.848 | 1.575 | 0.102±0.004 |
| **3** | 17.412±0.769 | 81.784±2.413 | 4.697 | 0.167±0.007 |
| **4** | 33.554±1.045 | 27.543±1.123 | 0.821 | 0.330±0.014 |
| **5** | 3.234±0.149 | 45.163±1.984 | 13.965 | 0.045±0.020 |
| **6** | 56.795±1.802 | 93.768±2.716 | 1.651 | 0.205±0.009 |
| **7** | 4.926±0.213 | 52.594±1.820 | 10.677 | 0.057±0.002 |
| **8** | 2.120±0.836 | 33.409±1.061 | 15.759 | 0.029±0.001 |
| **9** | 14.358±0.611 | 13.998±0.627 | 0.975 | 0.462±0.021 |
| **10** | >100 | >100 | - | 1.618±0.074 |
| **11** | >100 | >100 | - | 3.628±0.163 |
| **Doxorubicin** | 1.940±0.084 | >100 | >51.546 | - |
| **Letrozole** | 0.875±0.038 | >100 | >114.286 | 0.024±0.001 |

### References

[1] Berridge, M.V., Herst, P.M., Tan, A. S. 2005. "Tetrazolium dyes as tools in cell biology: new insights into their cellular reduction", Biotechnology Annual Review, 11, 127-152.
[2] Sa 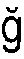 l 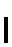 k, B.N., Ilg 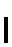 n, S., Ozkay, Y. 2016. "Synthesis of new donepezil analogues and investigation of their effects on cholinesterase enzymes", European Journal of Medicinal Chemistry, 124, 1026-1040.
[3] Osmaniye, D., Görgülü, 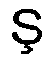 ., Sa 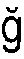 l 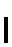 k, B.N., Levent, S., Ozkay, Y., Kaplancikli, Z.A. 2021. "Design, synthesis, *in vitro* and *in silico* studies of some novel thiazoledihydrofuran derivatives as aromatase inhibitors", Bioorganic Chemistry, 114, 105123.
[4] Osmaniye, D., Hidir, A., Sa 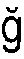 l 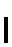 k, B.N., Levent, S., Ozkay, Y., Kaplanc 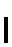 kl 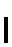 , Z.A. 2022. "Synthesis of new pyrimidine-triazole derivatives and investigation of their anticancer activities", Chemistry & Biodiversity, 19(8), e202200216.
[5] Osmaniye, D., Levent, S., Sa 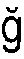 l 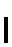 k, B.N., Karaduman, A.B., Ozkay, Y., Kaplanc 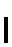 kl 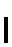 , Z.A. 2022. "Novel imidazole derivatives as potential aromatase and monoamine oxidase-B inhibitors against breast cancer", New Journal of Chemistry, 46(16), 7442-7451.
[6] Evren, A.E., Nuha, D., Dawbaa, S., Sa 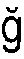 l 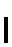 k, B.N., Yurtta 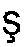 , L. 2022. "Synthesis of novel thiazolyl hydrazone derivatives as potent dual monoamine oxidase-aromatase inhibitors", European Journal of Medicinal Chemistry, 229, 114097.

## Claims

1. A thiazolo[5,4-*b*]pyridine derivative compound represented by Formula X for use in the treatment of breast cancer by reducing estrogen levels through aromatase inhibition, 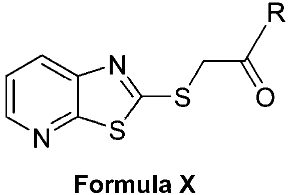 wherein the group represented by R is selected from among the following formulas:

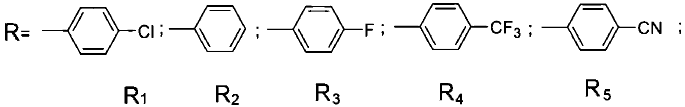

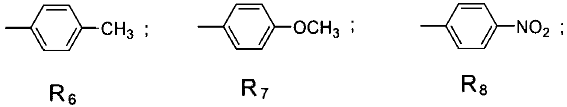

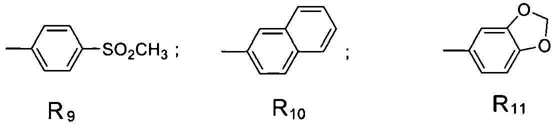

2. A compound according to claim 1 for use in the treatment of breast cancer, comprising one of the following formulas:
• 1-(4-chlorophenyl)-2-(thiazolo[5,4-*b*]pyridin-2-ylthio)ethan-1-one (Compound 1), or
• 1-phenyl-2-(thiazolo[5,4-*b*]pyridin-2-ylthio)ethan-1-one (Compound 2), or
• 1-(4-fluorophenyl)-2-(thiazolo[5,4-b]pyridin-2-ylthio)ethan-1-one (Compound 3), or
• 1-(4-trifluoromethylphenyl)-2-(thiazolo[5,4-*b*]pyridin-2-ylthio)ethan-1-one (Compound 4), or
• 1-(4-cyanophenyl)-2-(thiazolo[5,4-*b*]pyridin-2-ylthio)ethan-1-one (Compound 5), or
• 1-(4-methylphenyl)-2-(thiazolo[5,4-*b*]pyridin-2-ylthio)ethan-1-one (Compound 6), or
• 1-(4-methoxyphenyl)-2-(thiazolo[5,4-*b*]pyridin-2-ylthio)ethan-1-one (Compound 7), or
• 1-(4-nitrophenyl)-2-(thiazolo[5,4-*b*]pyridin-2-ylthio)ethan-1-one (Compound 8), or
• 1-(4-methylsulfonylphenyl)-2-(thiazolo[5,4-*b*]pyridin-2-ylthio)ethan-1-one (Compound 9), or
• 1-(naphthalen-2-yl)-2-(thiazolo[5,4-*b*]pyridin-2-ylthio)ethan-1-one (Compound 10), or
• 1-(1,3-benzodioxol-5-yl)-2-(thiazolo[5,4-*b*]pyridin-2-ylthio)ethan-1-one (Compound 11).

3. A compound according to claim 1 or claim 2 for use in the treatment of breast cancer, wherein it is a compound represented by Formula 1 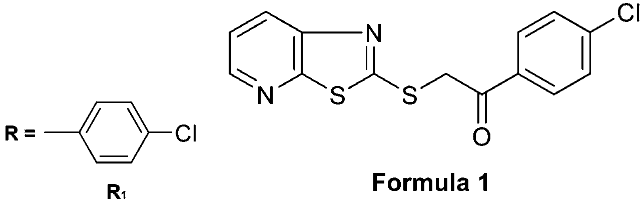 when R = R₁ ,
wherein the IC₅₀ value of 1-(4-chlorophenyl)-2-(thiazolo[5,4-*b*]pyridin-2-ylthio)ethan-1-one (Compound 1) is 45.289±1.962 µM for NIH/3T3 cells, 3.741±0.174 µM for MCF-7 cells, and 0.034±0.001 µM for aromatase inhibition.

4. A compound according to claim 1 or claim 2 for use in the treatment of breast cancer, wherein it is a compound represented by Formula 2 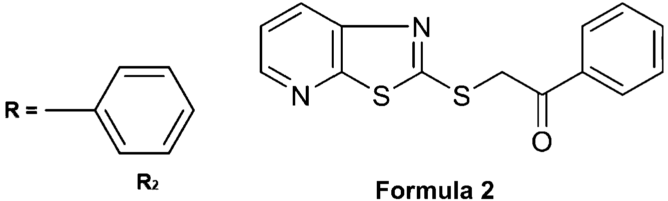 when R = R₂ ,
wherein the IC₅₀ value of 1-phenyl-2-(thiazolo[5,4-*b*]pyridin-2-ylthio)ethan-1-one (Compound 2) is 21.980±0.848 µM for NIH/3T3 cells, 13.956±0.512 µM for MCF-7 cells, and 0.102±0.004 µM for aromatase inhibition.

5. A compound according to claim 1 or claim 2 for use in the treatment of breast cancer, wherein it is a compound represented by Formula 3 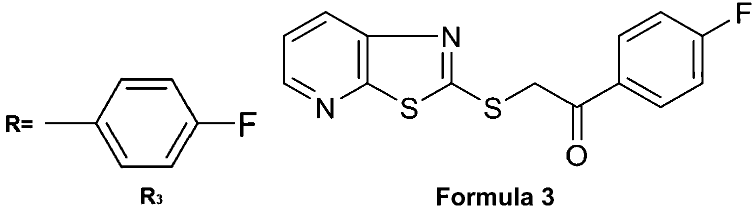 when R = R₃ ,
wherein the IC₅₀ value of 1-(4-fluorophenyl)-2-(thiazolo[5,4-*b*]pyridin-2-ylthio)ethan-1-one (Compound 3) is 81.784±2.413 µM for NIH/3T3 cells, 17.412±0.769 µM for MCF-7 cells, and 0.167±0.007 µM for aromatase inhibition.

6. A compound according to claim 1 or claim 2 for use in the treatment of breast cancer, wherein it is a compound represented by Formula 4 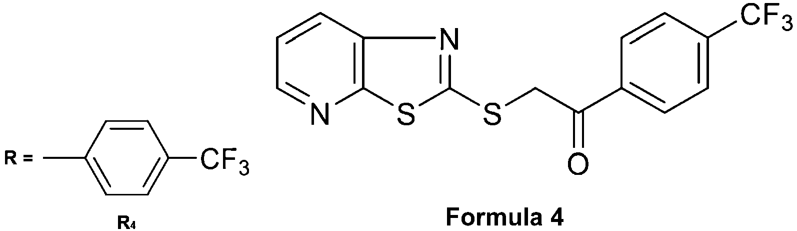 when R = R₄ ,
wherein the IC₅₀ value of 1-(4-trifluoromethylphenyl)-2-(thiazolo[5,4-*b*]pyridin-2-ylthio)ethan-1-one (Compound 4) is 27.543±1.123 µM for NIH/3T3 cells, 33.554±1.045 µM for MCF-7 cells, and 0.330±0.014 µM for aromatase inhibition.

7. A compound according to claim 1 or claim 2 for use in the treatment of breast cancer, wherein it is a compound represented by Formula 5 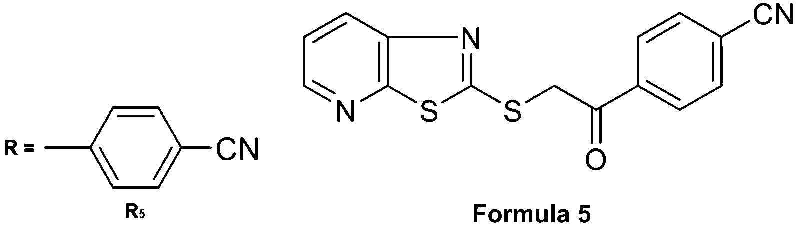 when R = R₅ ,
wherein the IC₅₀ value of 1-(4-cyanophenyl)-2-(thiazolo[5,4-*b*]pyridin-2-ylthio)ethan-1-one (Compound 5) is 45.163±1.984 µM for NIH/3T3 cells, 3.234±0.149 µM for MCF-7 cells, and 0.045±0.020 µM for aromatase inhibition.

8. A compound according to claim 1 or claim 2 for use in the treatment of breast cancer, wherein it is a compound represented by Formula 6 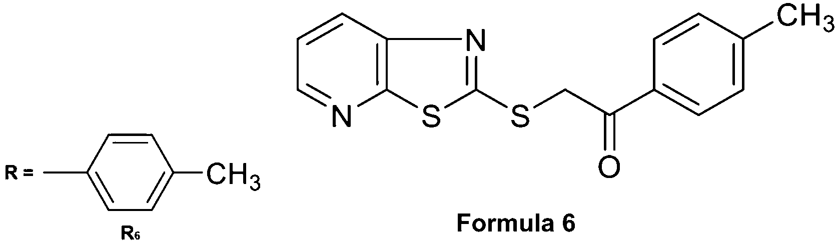 when R = R₆ ,
wherein the IC₅₀ value of 1-(4-methylphenyl)-2-(thiazolo[5,4-*b*]pyridin-2-ylthio)ethan-1-one (Compound 6) is 93.768±2.716 µM for NIH/3T3 cells, 56.795±1.802 µM for MCF-7 cells, and 0.205±0.009 µM for aromatase inhibition.

9. A compound according to claim 1 or claim 2 for use in the treatment of breast cancer, wherein it is a compound represented by Formula 7 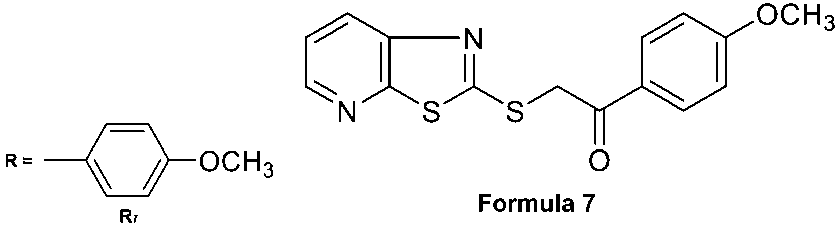 when R = R₇ ,
wherein the IC₅₀ value of 1-(4-methoxyphenyl)-2-(thiazolo[5,4-*b*]pyridin-2-ylthio)ethan-1-one (Compound 7) is 52.594 ± 1.820 µM for NIH/3T3 cells, 4.926 ± 0.213 µM for MCF-7 cells, and 0.057 ± 0.002 µM for aromatase inhibition.

10. A compound according to claim 1 or claim 2 for use in the treatment of breast cancer, wherein it is a compound represented by Formula 8 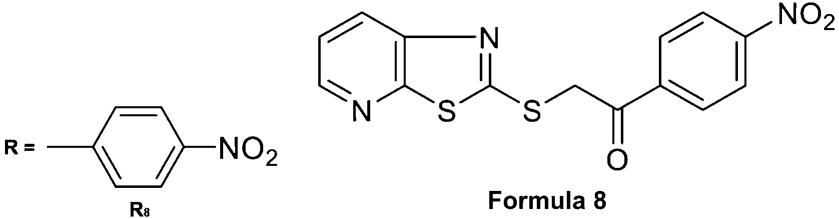 when R = R₈ ,
wherein the IC₅₀ value of 1-(4-nitrophenyl)-2-(thiazolo[5,4-*b*]pyridin-2-ylthio)ethan-1-one (Compound 8) is 33.409 ± 1.061 µM for NIH/3T3 cells, 2.120 ± 0.836 µM for MCF-7 cells, and 0.029 ± 0.001 µM for aromatase inhibition.

11. A compound according to claim 1 or claim 2 for use in the treatment of breast cancer, wherein it is a compound represented by Formula 9 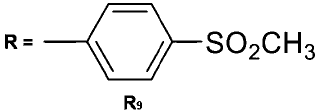 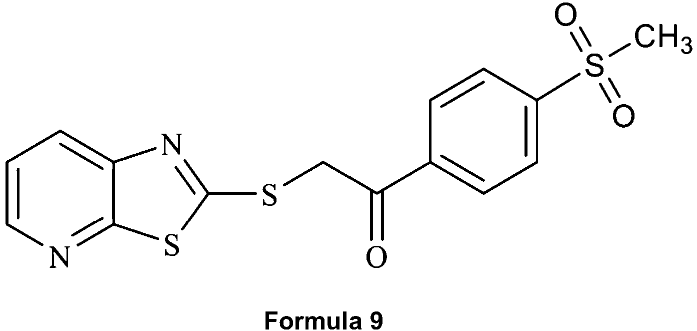 when R = R₉ ,
wherein the IC₅₀ value of 1-(4-methylsulfonylphenyl)-2-(thiazolo[5,4-*b*]pyridin-2-ylthio)ethan-1-one (Compound 9) is 13.998 ± 0.627 µM for NIH/3T3 cells, 14.358 ± 0.611 µM for MCF-7 cells, and 0.462 ± 0.021 µM for aromatase inhibition.

12. A compound according to claim 1 or claim 2 for use in the treatment of breast cancer, wherein it is a compound represented by Formula 10 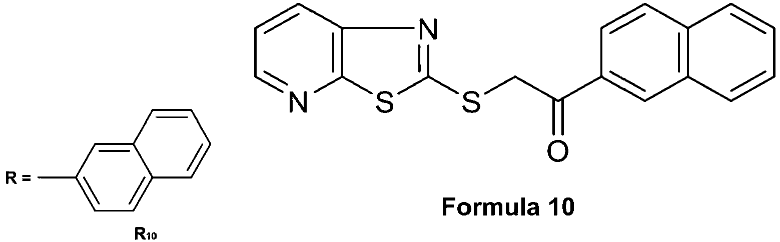 when R = R₁₀ ,
wherein the IC₅₀ value of 1-(naphthalen-2-yl)-2-(thiazolo[5,4-*b*]pyridin-2-ylthio)ethan-1-one (Compound 10) for aromatase inhibition is 1.618 ± 0.074 µM.

13. A compound according to claim 1 or claim 2 for use in the treatment of breast cancer, wherein it is a compound represented by Formula 11 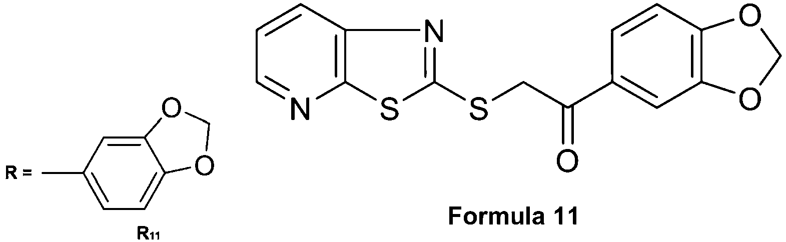 when R = R₁₁ ,
wherein the IC₅₀ value of 1-(1,3-benzodioxol-5-yl)-2-(thiazolo[5,4-*b*]pyridin-2-ylthio)ethan-1-one (Compound 11) for aromatase inhibition is 3.628 ± 0.163 µM.

14. A pharmaceutical composition for use in the treatment of breast cancer, comprising a compound according to any one of claims 1-79.

15. An anticancer drug for use in the treatment of breast cancer, comprising a compound according to any one of claims 1-79.
